(19)

Europäisches Patentamt

European Patent Office

Office européen des brevets

(11)  **EP 4 467 641 A1**

(12)  **EUROPEAN PATENT APPLICATION**

(43) Date of publication:
**27.11.2024  Bulletin 2024/48**

(21) Application number: **23203923.0**

(22) Date of filing: **16.10.2023**

(51) International Patent Classification (IPC):
***C12N 9/22*** *(2006.01)*     ***C12N 15/62*** *(2006.01)*

(52) Cooperative Patent Classification (CPC):
**C12N 9/22;** C07K 2319/80; C12N 9/78

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **25.05.2023  EP 23175382**

(71) Applicants:
 • **Universität Zürich**
  **8006 Zürich (CH)**
 • **ETH Zurich**
  **8092 Zürich (CH)**

(72) Inventors:
 • **Schmidheini, Lukas**
  **8006 Zürich (CH)**
 • **Schwank, Gerald**
  **8006 Zürich (CH)**

(74) Representative: **Schulz Junghans
Patentanwälte PartGmbB
Großbeerenstraße 71
10963 Berlin (DE)**

Remarks:
The complete document including Reference
Table(s) and the Sequence Listing(s) can be
downloaded from the EPO website

(54)  **CJCAS9 MUTANTS WITH RELAXED PAM COMPATIBILITY**

(57)      The invention relates to a *Cj*Cas9 variant characterized by at least one of the mutations E789K and S951G. The invention further relates to base editing enzymes comprising the Cas variant according to the invention and an adenosine deaminase activity, and to polynucleotides encoding same.

**EP 4 467 641 A1**

**Description**

Field

The present invention relates to variants of *Campylobacter jejuni* CAS9 variants having relaxed PAM requirements, and fusion proteins comprising same, as well as nucleic acids encoding such variants.

Background

Genome editing with RNA-guided programmable nucleases has transformed biomedical research and holds promise for therapeutic application in patients with genetic diseases. Cas9 endonucleases of the type II CRISPR immune defence system are the most widely used RNA-guided nucleases for genome editing. They are paired with programmable single guide (sg)RNAs, which contain a spacer sequence that targets the complementary protospacer on genomic DNA in case a downstream protospacer adjacent motif (PAM) is present. Binding of Cas9 endonucleases to target sites subsequently induces double strand (ds)DNA breaks, which lead to the installation of insertion/deletion (indel) mutations or precise editing from DNA donor templates via homologous recombination. More recently, the fusion of deaminases or reverse transcriptases (RTs) to catalytically impaired Cas9 led to the establishment of base editors (BEs) and prime editors (PEs). Importantly, these Cas9-based genome editing tools work independent of dsDNA break formation and homologous recombination and therefore enable precise editing in all cell types, including postmitotic primary cells.

Cas9 of *Streptococcus pyogenes* (SpCas9) was the first RNA-programmable endonuclease employed for genome editing, and due to its high activity and relatively simple PAM sequence (NGG) remains the most widespread Cas9 ortholog used to date. The large size of SpCas9 (1,368 aa), however, presents a challenge for efficient *in vivo* delivery, in particular if adeno-associated virus (AAV) vectors are used. AAVs are among the most promising nucleic acid delivery vehicles for the clinics, as they show low rates of genome integration and immunogenicity, and as they cover a broad range of tissue tropisms. Nevertheless, their limited cargo capacity of ~5 kb (including ITRs) prohibits the packaging of full-length *Sp*Cas9 together with its sgRNA on a single AAV vector. One possible solution to this problem is the replacement of *Sp*Cas9 with more compact Cas9 orthologues. Recent studies therefore employed Cas9 of *Staphylococcus aureus* (SaCas9 - 1,053 aa) and *Neisseria Meningitidis* (Nme2Cas9 - 1.080 aa) and packaged them as full-length nucleases or BEs together with their sgRNAs on single AAV vectors. Nevertheless, these constructs were still close to the packaging limit of AAV, and for BEs tissue-specific promoters had to be replaced by ubiquitous, minimal promoters (EF-1$\alpha$ or the nuclear RNA (U1a) promoter).

With 984 aa, *Campylobacter jejuni* (*Cj*)Cas9 is the smallest known Cas9 orthologue, making it a particularly attractive candidate for *in vivo* genome editing applications. However, similar to other smaller Cas9 orthologues, *Cj*Cas9 recognizes a relatively complex PAM sequence ($N_3$VRYAC), which occurs less frequently in the genome than the NGG motif of *Sp*Cas9. This strongly restrains the application of *Cj*Cas9, in particular for base- and prime editing where Cas9 has to bind DNA in a precisely defined distance to the targeted base.

Based on the above-mentioned state of the art, the objective of the present invention is to provide *Cj*CAS9 variants with lower PAM recognition stringency. This objective is attained by the subject-matter of the independent claims of the present specification, with further advantageous embodiments described in the dependent claims, examples, figures and general description of this specification.

*Sequences*

Sequences similar or homologous (e.g., at least about 85% sequence identity) to the sequences disclosed herein are also part of the invention.

Reference to identical sequences without specification of a percentage value implies 100% identical sequences (i.e. the same sequence).

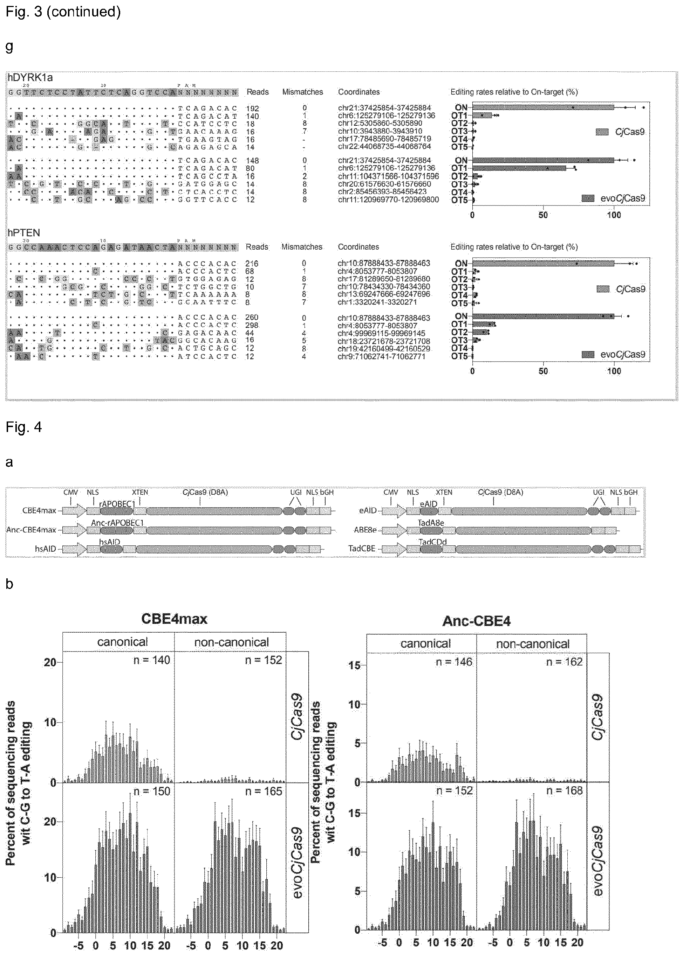

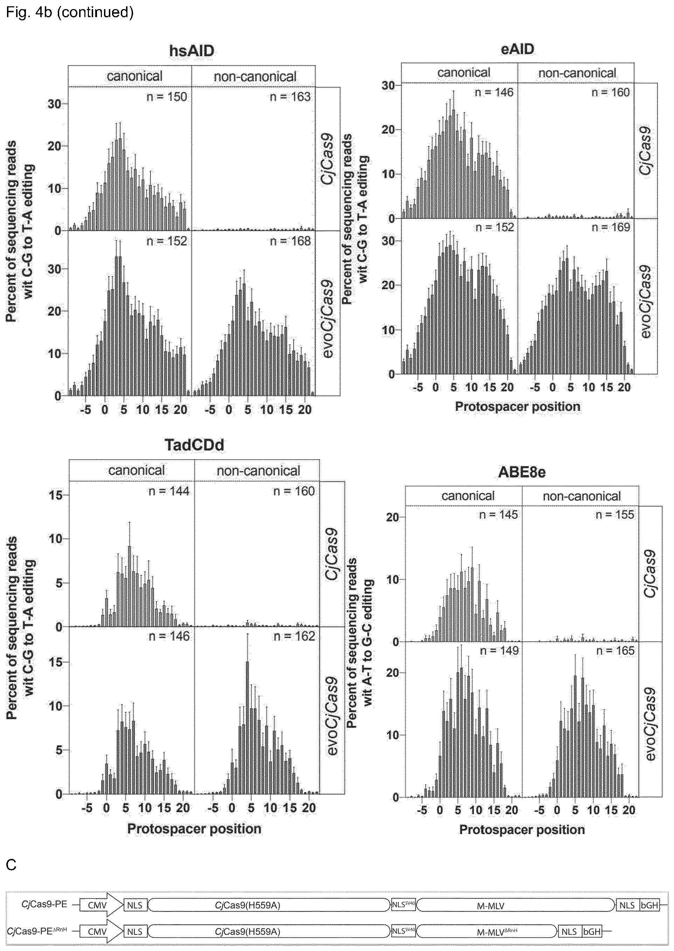

The term *having substantially the same biological activity* in the context of the present invention relates to either one or both main functions of a nucleic acid sequence editing protein, i.e. Indel, Base or Prime editing) in percent of edited sequence determined via High-throughput sequencing (HTS, see Figures 4b and the description relating thereto.

The term *homologue* in the context of the present specification relates to a functional polypeptide having a sequence identity of 85 % or more with SEQ ID NO 002.

The term *sgRNA* (single guide RNA) in the context of the present specification relates to an RNA molecule capable of sequence-specific repression of gene expression via the CRISPR (clustered regularly interspaced short palindromic repeats) mechanism.

The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

Detailed Description of the Invention

The invention generally relates to polypeptide sequences derived of *Campylobacter jejuni* Cas9 comprising one mutation selected from E789K and S951G, or both E789K and S951G, the numeration following the numeration of SEQ ID NO 001.

A first aspect of the invention relates to a *Campylobacter jejuni* Cas9 (*Cj*Cas9) variant, particularly a *Cj*Cas variant described by SEQ ID NO 001, which is characterized by the mutations E789K.

An alternative first aspect of the invention relates to a *Cj*Cas9 variant, particularly a *Cj*Cas variant described by SEQ ID NO 001, which is characterized by the mutations S951G.

In certain particular embodiments, the *Cj*Cas9 variant according to the invention comprises both E789K and S951G.

The position of the mutation is calculated relative to its position in SEQ ID NO 001, i.e. if a homologue with substantially identical sequence, but shorter or longer N-terminal sequence is used, the given numeration refers to the aligning positions of SEQ ID NO 001. The *Cj*Cas9 variant may be identical in all other positions to SEQ ID NO 001. Alternatively, the *Cj*Cas9 variant may comprise one, two, three or more of the mutations further described herein, and may otherwise be may be identical in all other positions to SEQ ID NO 001. Yet alternatively, the *Cj*Cas9 variant may be only equal or more than ($\geq$) 85%, $\geq$90%, $\geq$92%, %, $\geq$95%, $\geq$96%, %, $\geq$97%, $\geq$98%, identical to SEQ ID NO 001, and comprise at least one, particularly both of the mutations E789K and S951G, and optionally further L58Y, N821K, and D900K.

In addition to *Campylobacter jejuni* Cas9 orthologues, *Neisseria meningitidis* Cas9 orthologues share a high degree of identity with SEQ ID NO 001 and are expected to provide the same benefits as the sequence of CjCAS9-1 of SEQ ID NO 001 (see Chen et al, The CRISPR Journal Vol. 5(3); https://doi.org/10.1089/crispr.2021.0143.

Any of the Cas variants disclosed herein may be associated to a sgRNA molecule guiding its activity to a specific sequence.

*D900*

In certain embodiments, the *Cj*Cas9 variant according to the invention further comprises a mutation D900K or D900R. In certain particular embodiments, the *Cj*Cas9 variant further comprises the mutation D900K. This is described in WO2022045169A1 (Univ Tokyo; priority date 25 Aug 2020; also Nakagawa et al., Communications Biology Vol. 5, Article number: 211 (2022)).

In certain embodiments, the *Cj*Cas9 variant according to the invention comprises the mutations E789K and D900K.

In certain embodiments, the *Cj*Cas9 variant according to the invention comprises the mutations S951G and D900K.

In certain particular embodiments, the *Cj*Cas9 variant according to the invention comprises both E789K and S951G, and D900K.

*L58*

In certain embodiments, the *Cj*Cas9 variant according to the invention further comprises a substitution of L58 by Y or Q, particularly L58Y.

The inventors found that the evolution described in the examples also produced L58Q. The most favourable results in later testing were delivered by L58Y. The authors of the publication that first cites this position as interesting (Nakagawa et al., ibid.) suggest substituting leucine at amino acid number 58 by one of tyrosine, tryptophan, phenylalanine, or isoleucine.

In certain embodiments, L58 is substituted by an amino acid selected from the group consisting of tyrosine, tryptophan, phenylalanine, and isoleucine. In certain particular embodiments, L58 is substituted by tyrosine.

In certain embodiments, the *Cj*Cas9 variant according to the invention comprises the mutations E789K and L58Y.

In certain embodiments, the *Cj*Cas9 variant according to the invention comprises the mutations S951G and L58Y.

In certain particular embodiments, the *Cj*Cas9 variant according to the invention comprises both E789K and S951G, and L58Y.

In certain embodiments, the *Cj*Cas9 variant according to the invention comprises the mutations E789K, L58Y and D900K.

In certain embodiments, the *Cj*Cas9 variant according to the invention comprises the mutations S951G, L58Y and D900K.

In certain particular embodiments, the *Cj*Cas9 variant according to the invention comprises both E789K and S951G, L58Y and D900K.

*N821*

In certain embodiments, the *Cj*Cas9 variant according to the invention further comprises a substitution of N821, particularly N821K.

In certain embodiments, the *Cj*Cas9 variant according to the invention comprises the mutations E789K and N821K.

In certain embodiments, the *Cj*Cas9 variant according to the invention comprises the mutations S951G and N821K.

In certain particular embodiments, the *Cj*Cas9 variant according to the invention comprises both E789K and S951G, and N821K.

In certain embodiments, the *Cj*Cas9 variant according to the invention comprises the mutations E789K, L58Y and N821K.

In certain embodiments, the *Cj*Cas9 variant according to the invention comprises the mutations S951G, L58Y and N821K.

In certain particular embodiments, the *Cj*Cas9 variant according to the invention comprises both E789K and S951G, L58Y and N821K.

In certain embodiments, the *Cj*Cas9 variant according to the invention comprises the mutations E789K, D900K and N821K.

In certain embodiments, the *Cj*Cas9 variant according to the invention comprises the mutations S951G, D900K and N821K.

In certain particular embodiments, the *Cj*Cas9 variant according to the invention comprises both E789K and S951G, D900K and N821K.

In certain embodiments, the *Cj*Cas9 variant according to the invention comprises the mutations E789K, L58Y, D900K and N821K.

In certain embodiments, the *Cj*Cas9 variant according to the invention comprises the mutations S951G, L58Y, D900K and N821K.

In certain particular embodiments, the *Cj*Cas9 variant according to the invention comprises both E789K and S951G, L58Y, D900K and N821K.

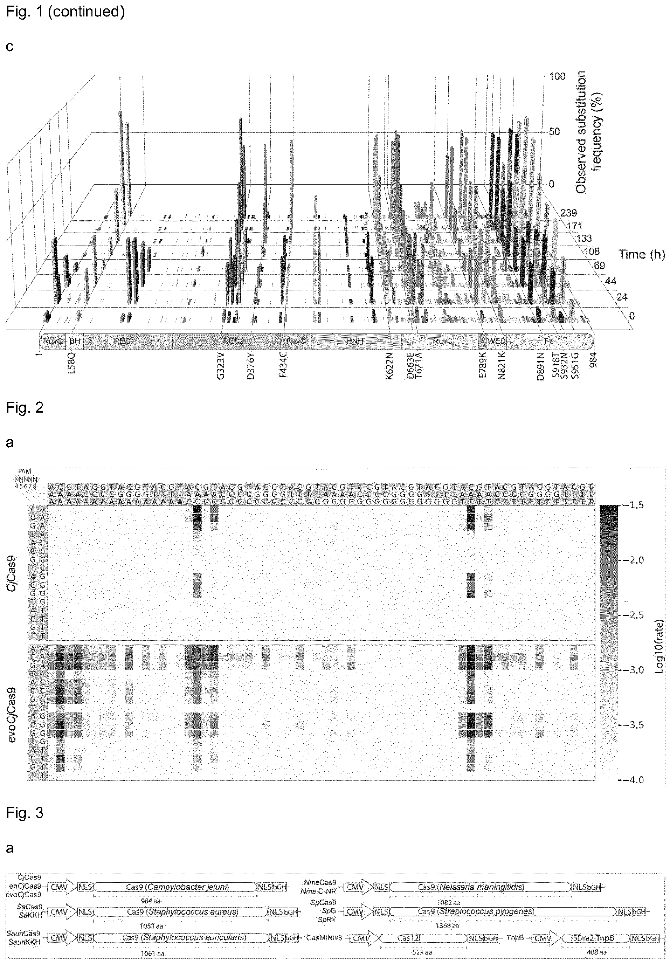

Fig. 2 shows the improvement, relative to the WT sequence, of the variant comprising a full set of muations (L58Y, E789K, N821K, D900K, S951G). Single mutations tested for PAM relaxation that resulted in a significant effect included E789K, E871K, D891N, E946K and S951G, which all resulted in PAM relaxation. The effect was particularly significant for a single E789K or S951G mutation (data not shown).

In certain embodiments, the *Cj*Cas9 variant according to the invention comprising any of the combinations recited above, further comprises one or more mutations selected from the group of G323V, D376Y, F434C, K622N, D663E and T671A.

The evolution performed in arriving at the present invention selected for mutations beneficial in CRISPR activation (CRISPRa). This is an application often used in research. Further work performed in the examples centred on Cas9 variants able to cut DNA (which is a function that cannot be screened in PACE). The mutations G323V, D376Y, F434C, K622N, D663E and T671A impacted nuclease activity, and thus were not further pursued. The inventors however do expect these mutations to be advantageous in applications where a nuclease deficient Cas9 is required (e.g. CRISPR activation & inhibition, epigenome editing).

In certain embodiments, the *Cj*Cas9 variant according to the invention comprising any of the combinations recited above, further comprises one or more mutations selected from the group of E871K, D891N and E946K.

In certain embodiments, the *Cj*Cas9 variant according to the invention comprises the sequence of SEQ ID NO 002, or a sequence having at least 95% identity to SEQ ID NO 002 and bears the mutations L58Y, E789K, N821K, D900K, S951G. The variant according to these embodiments defined by identity to SEQ ID NO 002 is characterized by a less stringent PAM requirement (defined as functional PAM sites in a random sequence) than SEQ ID NO 001.

A functional PAM can be defined as a PAM site that allows clearly distinguishable editing efficiency (comprising at least 50% efficiency of a protein most efficiently recognized PAM).

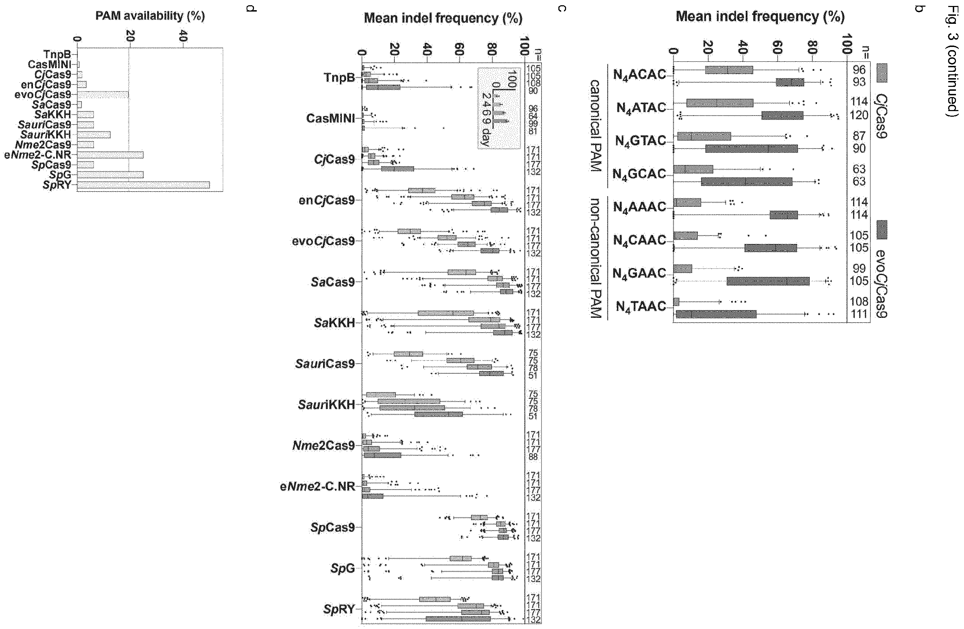

PAM stringency can be indirectly quantified by calculating the probability of PAM occurrence within a perfectly random DNA sequence of length PAM and considering only one strand direction (see Example 1). This parameter is quantified in Fig. 3d as PAM availability in %. For example, an NGN PAM would correspond to 25%. For evoCjCas9, the inventors determined this value to be 19.34%. For further information, see the caption of Fig. 3d and Example 1.

The determination whether a potential PAM sequence is actually recognized by the protein is a parameter for which different methods are available. The value for this parameter used in the context of this specification (at least 50% efficiency compared to the most active PAM site of a Cas9) may be determined experimentally in a number of ways (e.g. HT-PAMDA or HTS (both were performed in our manuscript). The method described in Example 1 is used when in doubt.

Once it has been defined when a PAM sequence is considered as recognized, one can (mathematically) determine a value for PAM relaxation. The inventors quantified this as the probability of PAM occurrence within a (perfectly) random (ss)DNA sequence of length PAM.

*Editing enzymes*

Another aspect of the invention relates to a polypeptide comprising a CjCAS9 variant according to any one of the preceding embodiments of the first aspect of the invention, wherein the polypeptide further comprises a nucleotide deaminase activity.

In certain embodiments, the deaminase is an adenosine deaminase activity.

A great number of deaminases offer themselves for linking to the CAS9 variant of the invention to construct a base editing enzyme. The inventors hypothesize, without wanting to be bound by theory, that deaminases characterized by a high kinetic rate of activity may have a favourable effect in combining with the CAS9 variant of the invention, as a higher kinetic rate is expected to compensate for the presumably shorter residency time resulting from the reduction in PAM stringency.

In certain embodiments, the deaminase is tadA, an essential tRNA-specific adenosine deaminase from *Escherichia coli.*

In certain embodiments, the deaminase is tadA 7.10, an evolved essential tRNA-specific adenosine deaminase from *Escherichia coli,* see Richter et al., Nat Biotechnol. 2020 Jul; 38(7): 883-891.

In certain embodiments, the deaminase is tadA 8, an evolved essential tRNA-specific adenosine deaminase from *Escherichia coli,* see Yan, Molecular Plant 14, 722-731 (2021).

In certain embodiments, the deaminase is a cytidine deaminase activity.

In certain embodiments, the deaminase is APOBEC1, a human cytidine deaminase (Uniprot P41238).

In certain embodiments, the deaminase is TadCBEd, a human cytidine deaminase evolved from tadA. See Neugebauer et al. Nat Biotechnol (2022). https://doi.org/10.1038/s41587-022-01533-6.

In certain embodiments, the polypeptide comprising a CjCAS9 variant according to the preceding aspect further comprises a reverse transcriptase activity.

In certain embodiments, the polypeptide comprising a CjCAS9 variant according to the preceding aspect does not comprise an RNAseH activity.

*Polynucleotide sequences*

Another aspect of the invention relates to a polynucleotide sequence encoding a *CJ*Cas9 variant or a polypeptide comprising a *Cj*CAS9 variant, as specified in any one of the preceding aspects and embodiments.

The polynucleotide sequence according to this aspect of the invention may be a DNA sequence.

The DNA sequence may be a "naked" DNA sequence, for example an expression plasmid sequence or a microchromosome. The DNA sequence may alternatively be a DNA viral sequence.

In other embodiments, the polynucleotide sequence according to this aspect of the invention may be a RNA sequence.

The RNA sequence may be a "naked" RNA sequence, for example an mRNA sequence comprised of "natural" or synthetic RNA. The RNA sequence may alternatively be a RNA viral sequence.

The polynucleotide sequence encoding a *CJ*Cas9 variant may be present in a recombinant cell under control of a promoter sequence operable in that cell to provide transgene expression of the variant or editing enzyme that the variant may be part of. The

The polynucleotide sequence encoding a *CJ*Cas9 variant may be present in a viral vector capable of specifically entering a target cell to deliver the variant or editing enzyme that the variant may be part of.

Another aspect of the invention relates to a nucleic acid expression vector comprising a polynucleotide sequence according to the above specification under control of a promoter operable in a target cell. In particular embodiments, the expression vector is an AAV virus particle.

In a more particular embodiment, the expression vector is an AAV9 virus particle.

The inventors illustrated the invention in AAV variant AAV9, which in their hands was easily produced and efficient in the experiments performed. The skilled person is aware how to select an AAV variant based on its specificity for a particular target tissue. The inventors predict that the invention can be easily practiced in any AAV variant available depending on the application at hand.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

## Description of the Figures

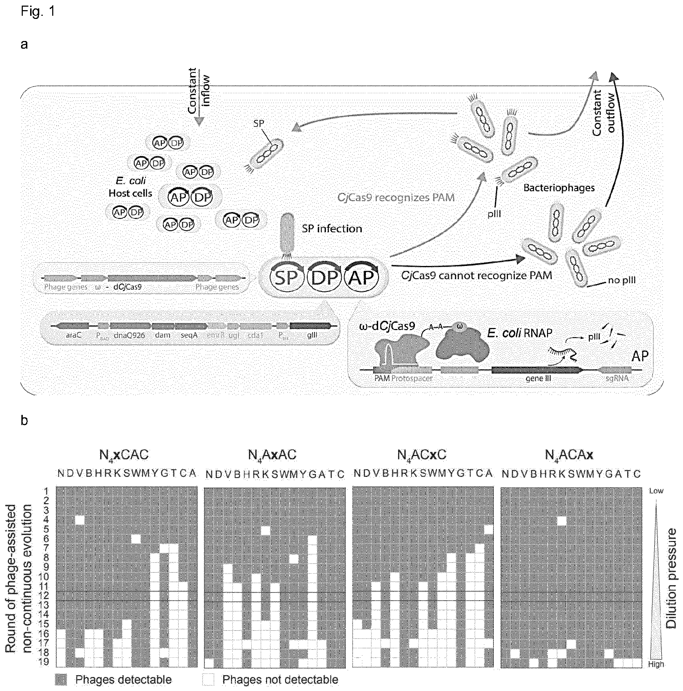

**Fig. 1 | Phage-assisted continuous and non-continuous evolution (PACE) to broaden the PAM compatibility of CjCas9.** a, Schematics of the PACE experiment. Selection phages (SP) infect *E. coli* host cells and use the hosts machinery to replicate and translate required phage genes. Instead of gene III, SP carries the ω-subunit-d*Cj*Cas9 fusion gene. Cas9 recognition of a PAM and protospacer sequence on the accessory plasmid (AP) allows recruitment of endogenous *E. coli* RNA polymerase via the ω-subunit and subsequent translation of gene III (pIII). Efficient recognition of the PAM and protospacer sequence results in infectious phages that are able to sustain in the lagoon, whereas weak or lack of recognition results in phage wash-out. The mutation rate during phage replication is controlled and elevated via arabinose-inducible expression of mutagenesis genes from a mutagenesis plasmid (DP6). b, Illustrative overview of 19 rounds of PANCE using accessory plasmids containing different PAM sites (according to IUPAC notation). Phages were increasingly diluted from one round to the next. Red indicates presence of phages, white indicates absence. c, Long-read sequencing of individual phages (n = 811) at 8 different time points during PACE. For each time point, the mutation frequency and amino acid position is shown. Amino acid positions substituted in more than 50% of phage genotypes are indicated.

**Figure 2 | Characterization of the PAM compatibility of evoCjCas9.** a, HT-PAMDA characterization of *Cj*Cas9 and evo*Cj*Cas9 illustrating their PAM preference at PAM positions 4 to 8. The $\log_{10}$ rate constant represents the mean of two replicates against two distinct spacer sequences. b, Protein structure of *Cj*Cas9 with evo*Cj*Cas9 mutations introduced and highlighted (left, based on PDB: 5X2H). Detailed view illustrating differences between *Cj*Cas9 (top panels) and evoCjCas9 (bottom panels) residues interacting with PAM nucleotides of the target strand.

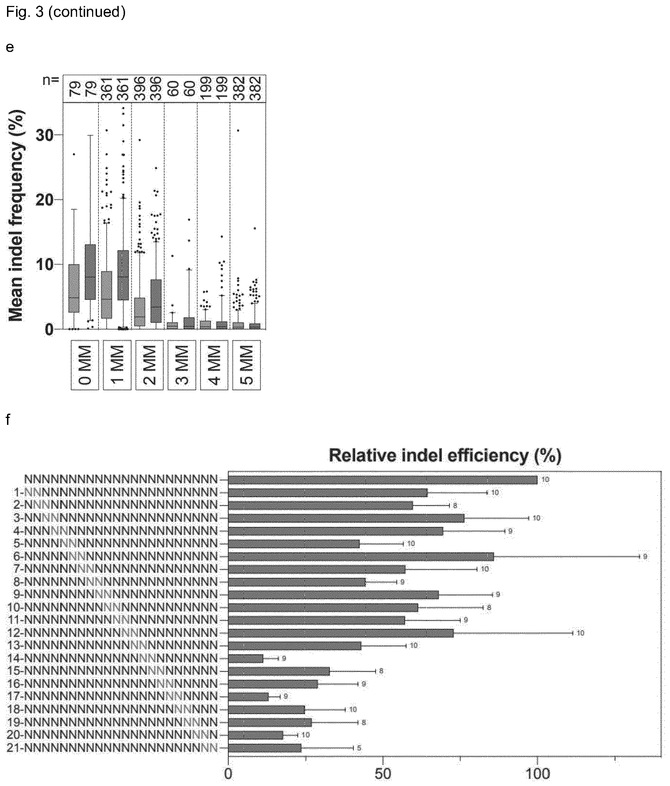

**Figure 3 | Indel formation rates of evoCjCas9 compared to CjCas9 and other commonly used RNA-guided endonucleases,** a, Illustration and size comparison of the expression vectors encoding for different Cas enzymes. b, Observed indel frequencies for *Cj*Cas9 and evo*Cj*Cas9 on 8 different PAM sites. c, Observed indel frequencies for different commonly used RNA-guided endonucleases and PAM relaxed Cas variants on different target sites with the most optimal PAM for each nuclease. Colors represent different timepoints. d, PAM frequency of the different RNA-guided endonucleases, illustrated as the probability of encountering a PAM on a randomly selected target site in a random DNA sequence (in %). Calculations are based on TTGAT for TnpB; TTTR for CasMINIv3.1; PAM sequences with a kinetic rate constant >$10^{-3}$ for *Cj*Cas9 variants; $N_2$GRRT for *Sa*Cas9; $N_3$RRT for SaKKH; $N_2$GG for *Sauri*Cas9; $N_2$RG for *Sauri*KKH; $N_4$CC for Nme2Cas9; $N_4$C for eNme2-C.NR; NGG for *Sp*Cas9; NG for *Sp*G and NR for *Sp*RY. e, Mean indel frequencies of *Cj*Cas9-variants at on-target sites (0 MM) and potential off-target sites containing up to 5 mismatches (MM). f, Indel frequencies of evo*Cj*Cas9 at sites with dinucleotide mismatches normalized to the on-target site. Dinucleotides highlighted in red represent mismatch positions. PAM at position 23-30. Number above bars indicate amount of different target sites. g, Relative indel frequencies evaluated by HTS at off-target sites detected by CHANGE-seq for two sgRNAs. Boxplots in (b, c, e) represent the 25th, 50th, and 75th percentiles. Whiskers indicate 5 and 95 percentiles. Numbers (n) above plots indicate number of datapoints. Bars in (f, g) represent mean, with error bars representing standard error of the mean. b, c, e, f, g, Indels refer to insertion, deletions, or substitutions. The sequences shown in panel g relate to the SEQ ID NOs: 28-53.

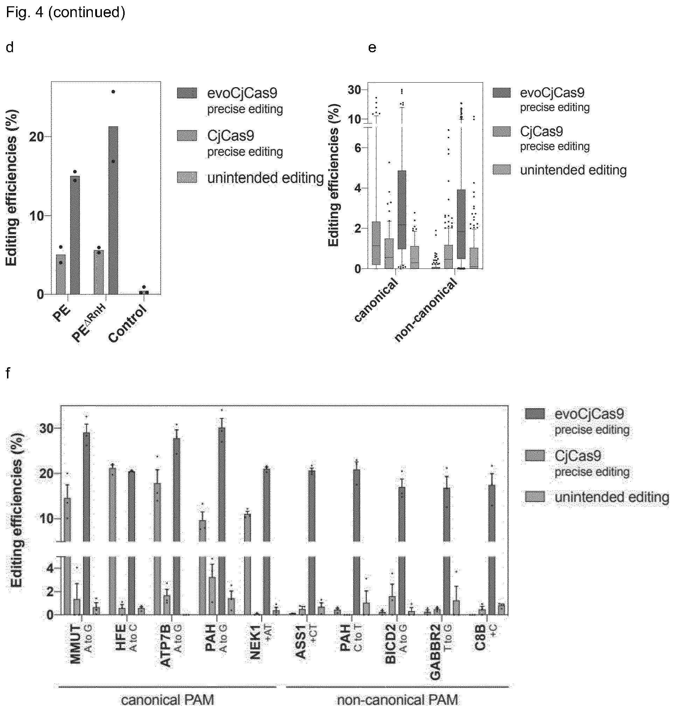

**Figure 4 | Base- and Prime editing with CjCas9 and evoCjCas9.** a, Illustration of expression vectors encoding for different CjCas9 BEs. b, Observed nucleotide transitions within the target sites with canonical and non-canonical PAMs. Bars represent mean of three independent biological replicates on $N_4$ACAC, $N_4$ATAC, $N_4$GTAC, and $N_4$GCAC (canonical) or $N_4$AAAC, $N_4$CAAC, $N_4$GAAC and $N_4$TAAC (non-canonical) PAM sites. Number of target sites (n) is indicated within each plot. Protospacer positions from -9 to 22 are shown. c, Illustration of expression vectors encoding for *Cj*Cas9 prime editors with the full length (top) or RnaseH depleted M-MLV reverse transcriptase (bottom). d, Comparison of *Cj*Cas9 or evo*Cj*Cas9 PEs installing an A to G transition at the AAVS1 locus. e, Mean editing efficiencies of *Cj*Cas9 or evo*Cj*Cas9-PE$^{\Delta RnH}$ on a selection of loci with canonical or non-canonical PAM sites. The loci of the target sites and type of edits are indicated. Bars (b, d, e) represent mean of biological replicates in HEK293T cells, with error bars indicating standard error of the mean. f, Mean prime editing efficiencies and indel rates on 64 (canonical) and 172 (non-canonical) integrated target sites. Boxplots represent the 25th, 50th, and 75th percentiles. Whiskers indicate 5 and 95 percentiles. *Cj*Cas9 in blue, evo*Cj*Cas9 in red (b, d - f).

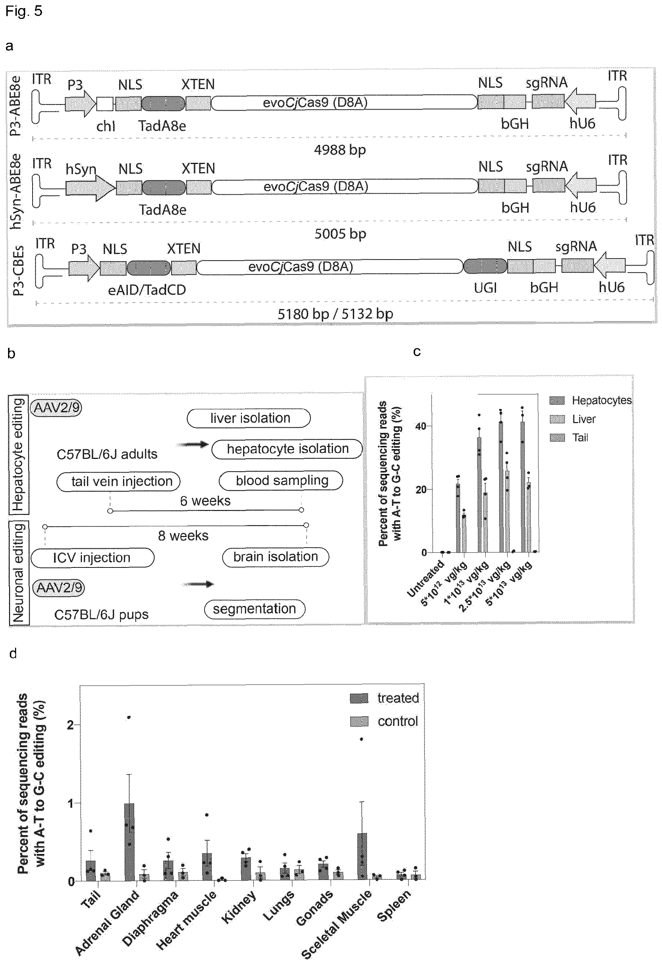

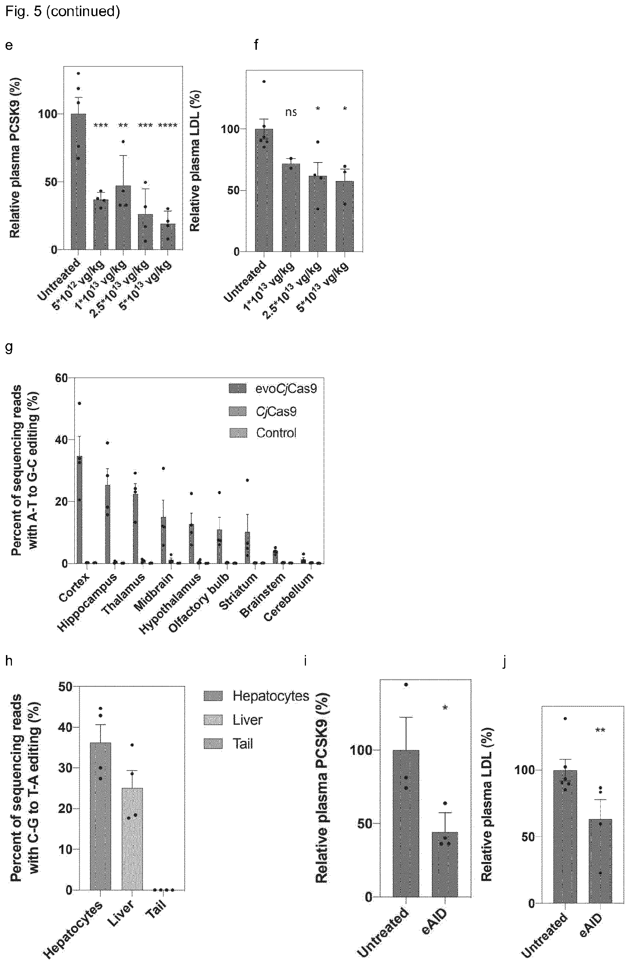

**Figure 5 | *In vivo* genome editing with compact evo*Cj*Cas9 adenine and cytosine BE.** a, Schematic representation of single AAV vectors used for evo*Cj*Cas9 base editing. Elements are not illustrated to scale. b, Illustration of the experimental workflow for *in vivo* adenine or cytosine base editing at the *Pcsk9* locus in the liver and of the *Gpr6* locus in the brain. c, d, A-to-G editing at the targeted *Pcsk9* splice site with different AAV concentrations, analyzed in isolated hepatocytes, whole liver samples, the tail (c, same legend as in h) and other tissues (d). e, Plasma PCSK9 levels relative to untreated control as determined by ELISA. ***$P$ = 0.0006, **$P$ = 0.0029, ***$P$ = 0.0001, ****$P$ < 0.0001 (left to right). f, Plasma LDL cholesterol levels. $P$ = 0.2461, *$P$ = 0.0278, *$P$ = 0.027 (left to right). g, A-to-G editing at the targeted site in the *Gpr6* locus (non-canonical CCGCCAAC PAM) in isolated brain tissues for *Cj*Cas9 and evo*Cj*Cas9. h, C-to-T editing at the targeted *Pcsk9* site with evo*Cj*Cas9 cytosine base editor (eAID) in isolated hepatocytes, whole liver samples and the tail. i, Plasma PCSK9 levels relative to untreated control as determined by ELISA. *$P$ = 0.0286. j, Plasma LDL cholesterol levels. **$P$ = 0.0095. Means were compared using one-way ANOVA with Dunnett correction (e,f) or one-tailed t-test (Mann-Whitney) (i, j) . Bars (c-j) represent mean with error bars representing standard error of the mean. Individual data points as black dots. vg, vector genomes, chI, chimeric Intron, ICV, intracerebroventricular.

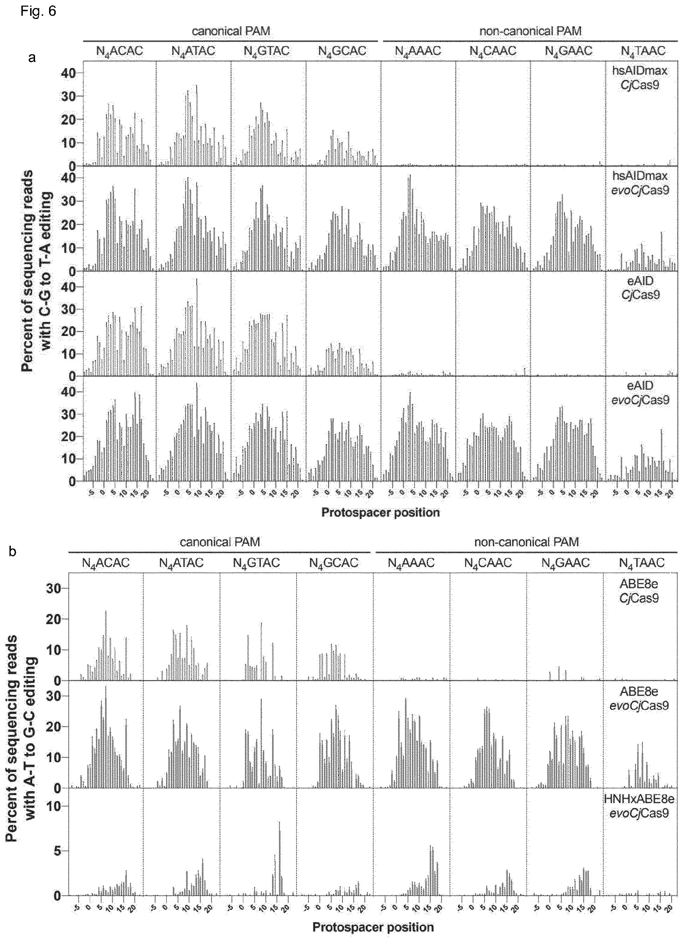

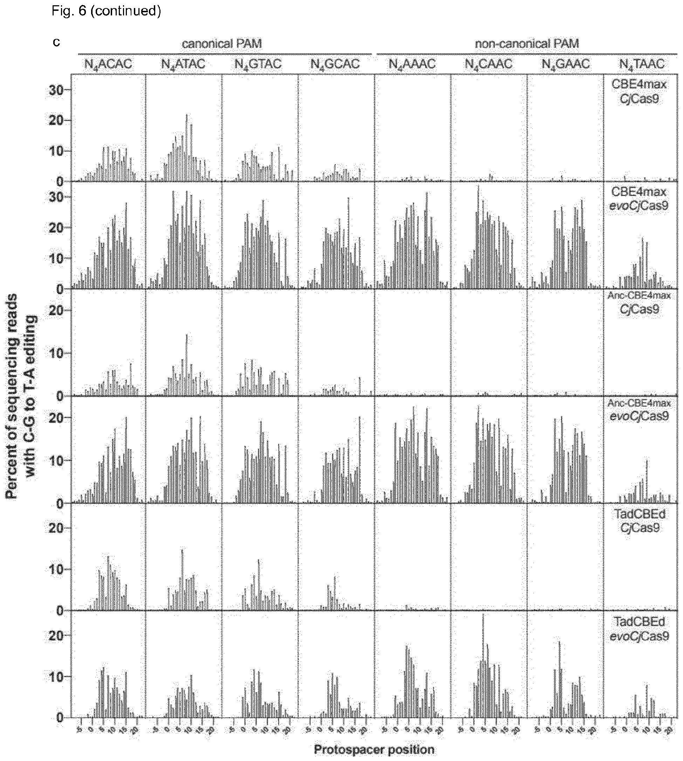

**Figure 6 | Base editing with *Cj*Cas9 and evo*Cj*Cas9.** Observed nucleotide transitions of adenine BEs (top) and cytosine BEs (bottom panel) with *Cj*Cas9 and evo*Cj*Cas9 on 8 different PAM sites (with >20 target sites per PAM and a total of 254 target sites for each architecture). The BE architecture is indicated in rightmost panels. Bars represent mean of three independent biological replicates, with error bars representing standard error of the mean.

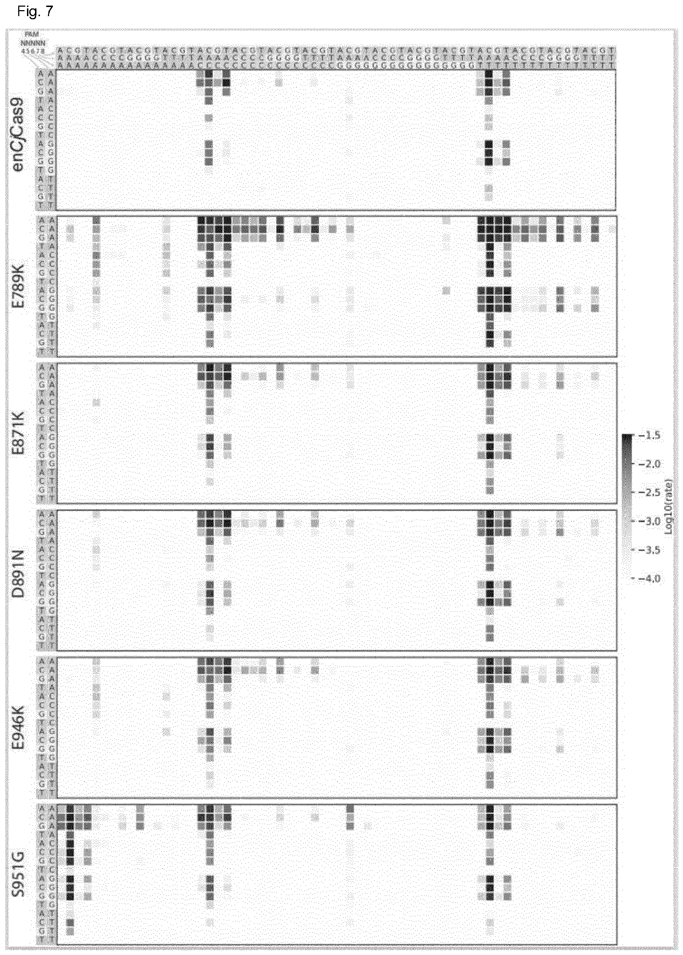

**Figure 7| Contribution of consensus mutations in *Cj*Cas9 on PAM relaxation.** HT-PAMDA characterization of en*Cj*Cas9 (top panel) with introduced PAM relaxing mutations (lower panels) illustrating their PAM preference at PAM positions 5 to 8. The $\log_{10}$ rate constant represents the mean of two replicates against two distinct spacer sequences.

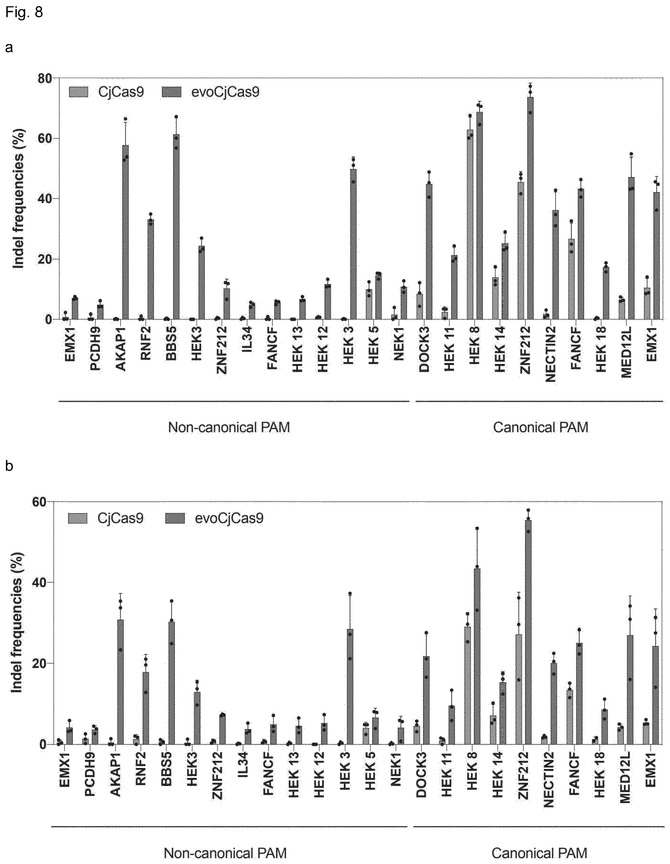

**Figure 8| Indel formation with *Cj*Cas9 and evo*Cj*Cas9 on endogenous target sites.** a, b, Indel frequencies of *Cj*Cas9 and evo*Cj*Cas9 on 14 non-canonical and 10 canonical PAM sites in HEK293T cells with selection harvested 9 days (a) or without selection 4 days (b) post transfection. Bars (a, b) represent mean with error bars representing standard error of the mean. Individual data points as black dots.

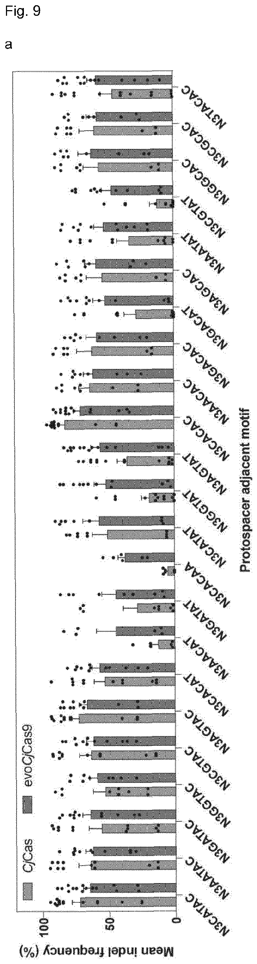

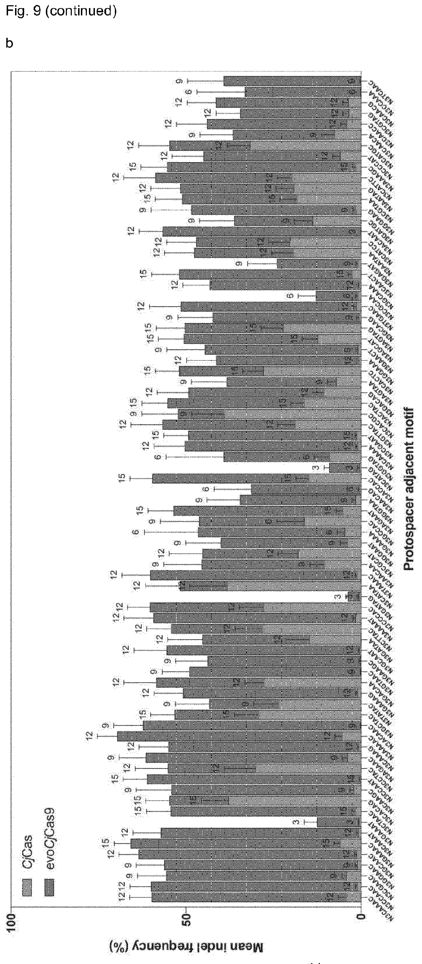

**Figure 9| Indel formation rates with *Cj*Cas9 and evo*Cj*Cas9 at target sites with different PAMs.** Mean indel frequency in self-targeting libraries on canonical (a) and non-canonical (b) PAM sites. Bars represent mean with error bars representing standard error of the mean, with individual data points of biological triplicates shown as black dots (a) or indicated above error bars (b). The *in vitro* PAMDA assay (Fig. 2a) revealed few motifs recognized by *Cj*Cas9 that lie outside of the simplified N$_3$VRYAC motif (e.g. N$_3$MACAN), albeit with low efficiency. We define sequences that fall outside of N$_3$VRYAC as non-canonical motifs for the purpose of simplification.

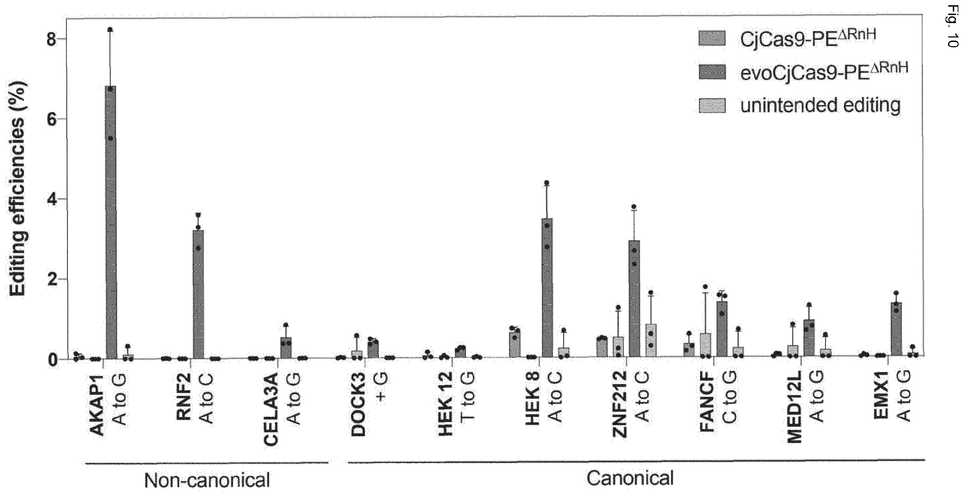

**Figure 10| Prime editing rates with *Cj*Cas9 and evo*Cj*Cas9 on endogenous target sites.** Prime editing and corresponding unintended editing frequencies of *Cj*Cas9 and evo*Cj*Cas9-PE$^{\Delta RnH}$ on 3 canonical and 7 non-canonical target sites in HEK293T cells. Bars represent mean with error bars representing standard error of the mean. Individual data points as black dots.

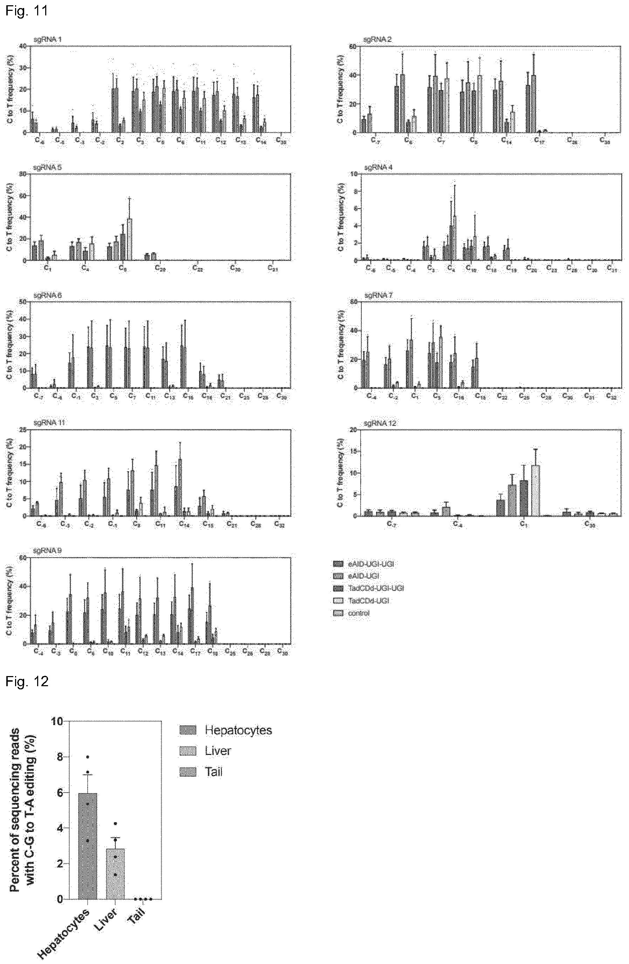

**Figure 11| Cytosine base editing with *Cj*Cas9 and evo*Cj*Cas9 on sites in the *Pcsk9* locus.** Editing frequency (C-to-T) with eAID or TadCDd CBEs with one or two C-terminal UGI domains in N2a cells. Bars represent mean with error bars representing standard error of the mean. Individual data points as black dots.

**Figure 12| *In vivo* genome editing with the evo*Cj*Cas9-TadCDd cytosine BE.** Observed editing at the targeted *Pcsk9* site with an AAV construct containing the evo*Cj*Cas9-TadCDd base editor. Bars represent mean with error bars representing standard error of the mean, with individual data points shown as black dots.

Examples

The examples show phage-assisted continuous directed evolution (PACE) to generate evo*Cj*Cas9. This *Cj*Cas9 variant obtained thereby recognizes $N_4AH$ and NsHA PAM sequences, which occur ten-times more frequently in the genome than the canonical $N_3VRYAC$ PAM sequence. evo*Cj*Cas9 also possesses a higher nuclease activity than wild type *Cj*Cas9 and enables robust base- and prime editing at canonical and non-canonical PAM sites. Proof-of-concept is provided for tissue-specific *in vivo* base editing with evo*Cj*Cas9 using a one-vector AAV system.

*Example 1: PAM recognition assay*

We define recognition of potential PAMs by performing high-throughput PAM detection assay as recently described (Walton et. al., 2021, Nat. Protoc. (https://doi.org/10.1038/s41596-020-00465-2)). In brief, Cas9 is cloned in pCMV-T7-SpCas9-P2A-EGFP (Addgene #139987) and expressed in HEK293T (ATCC CRL-3216) cells for 48 hours. Whole-cell lysates are collected and normalized to a concentration corresponding to 150 nM fluorescein dye. Target-specific sgRNAs are *in vitro* transcribed using HiScribe T7 High Yield RNA Synthesis Kit. Substrate libraries with different target sites and PAM libraries are cloned into p11-LacY-wtxq (Addgene #69056). gRNA (1.1 μM) and normalized cell lysate (83 nM fluorescein) are complexed for 10 minutes at 37 °C. The ribonucleoprotein mixture (0.5 μM gRNA, 37.5 nM fluorescein lysate) is added to the substrate library (2.5 nM) and the reaction is stopped at different time intervals (1, 8, and 32 minutes). For all time points, substrate libraries are individually PCR amplified using time point-specific barcodes. Samples are pooled and sequenced on a high-throughput sequencing platform (e.g. Illumina). A Cas9 protein is characterized on two different target sites, with the PAM library spanning positions 4 to 8 (Target 1 and Target 2). Two replicates for each target sequence are performed on different days, and sequencing data is processed using the published python script (Walton et. al., 2021, Nat. Protoc.). Derived depletion rate constants for each PAM are averaged. We define a Cas9 to exhibit activity towards a PAM when the depletion rate constant is equal to or larger than 0.001.

Based on the number of PAMs a Cas9 recognizes, the PAM availability in % is calculated as follows (see Fig. 3d):

$$\text{(number of PAMs a Cas9 exhibits activity on)} / \text{(number of possible PAMs (= 4 to the power of total possible PAM positions)} * 100$$

At the example of evoCjCas9: $198/(4^5)*100 = 19.33\%$.

At the example of wild-type CjCas9: $19/(4^5)*100 = 1.86\%$

At the example of enCjCas9: $35/(4^5)*100 = 3.42\%$

At the example of SpCas9 (assuming NGG PAM being recognized): $4/(4^3)*100 = 6.25\%$

Alternatively, PAM recognition can be expressed as a parameter describing every how many bases in a random DNA sequence a Cas9 would encounter a PAM on which it exhibits activity:

(number of possible PAMs) / (number of PAMs a Cas9 exhibits activity on)

At the example of evoCjCas9: $(4^5)/198 = 5.17$

At the example of wild-type CjCas9: $(4^5)/19 = 53.89$

At the example of enCjCas9: $(4^5)/35 = 29.26$

At the example of SpCas9 (assuming NGG PAM being recognized): $(4^3)/4 = 16$

*Example 2: Results*

*Phage-assisted continuous and non-continuous directed evolution of CjCas9*

PACE is a technique for directed protein evolution, where the gene of interest is continuously mutated and transferred from bacterial host cell to host cell via a modified M13 filamentous bacteriophage in an activity of interest-dependent manner. In our PACE setup the essential gene III on the M13 selection phage (SP) is replaced by a catalytically dead *Cj*Cas9 (d*Cj*Cas9), which is C-terminally fused to the ω subunit of bacterial RNA polymerase. SP infection of *E. coli* host cells carrying the accessory plasmid (AP) results in complexion of d*Cj*Cas9 with an sgRNA that directs the complex to the promoter region of gene III (**Fig. 1a**). Recognition of the protospacer and PAM sequence leads to stable binding of the d*Cj*Cas9-ω complex, recruitment of the RNA polymerase and subsequent expression of gene III. This completes the life cycle of the phage and enables the assembly of functional and infectious phage particles. The additional presence of the drift plasmid (DP) in *E. coli* host cells ensures mutagenesis of the phage genome during replication, and continuous addition and draining of media with fresh host cells leads to a steady phage dilution and sets the evolutionary pressure. By installing a library of non-canonical PAM sequences on the AP, we further ensured linkage of phage propagation to d*Cj*Cas9 variants that recognize these novel PAMs in our setup (**Fig. 1a**). Since the first four positions of the canonical *Cj*Cas9 PAM show only minor nucleotide preferences (NNNV), we focused our efforts on the relaxation of the last 4 PAM positions (RYAC). Our first attempt to start PACE with a PAM library that is fully randomized at these 4 positions, however, was not successful as the selection was too stringent and phages could not be propagated (determined by RT-qPCR; CT > 30). Therefore, we next tried to pre-select *Cj*Cas9 variants with relaxed PAM recognition using phage-assisted non-continuous evolution (PANCE). In this setup we used the same evolution logic, but reduced the dilution pressure by propagating phages via serial phage dilution instead of dilution in a continuous flow. However, also the moderate dilution rates used in PANCE resulted in infrequent gene III activation and loss of phages, as determined by RT-qPCR (CT > 30). To reduce stringency further, we next performed PANCE in an arrayed fashion where we individually replaced nucleotides at PAM positions 5-8 on the AP in all possible combinations (15 different nucleotide compositions per position, resulting in 56 different combinations plus 4 canonical $N_4$ACAC PAMs - **Fig. 1b**). In this setup we identified phages to withstand 200-fold dilution steps for PAM combinations close to the canonical PAM, while more unfavoured PAM sequences (e.g. T, G and C at positions 5, 6 and 7, respectively) led to a phage wash-out unless the AP was mixed with a fraction of plasmids with canonical PAM nucleotides (**Fig. 1b**). Interestingly, lagoons with all nucleotide variations at position 8 supported phage propagation, while positions 6 and 7 possessed more stringent PAM requirements (**Fig. 1b**). After 12 rounds of dilution (a total of $5*10^{23}$-fold dilution), 45 lagoons maintained detectable phage genomes. These were mixed in a 1:1 ratio with wild-type ω-d*Cj*Cas9-phages and used to seed a lagoon of a PACE apparatus with a continuous inflow of fresh host cells containing an AP with a randomized PAM library for positions 6-8. The pre-relaxation with PANCE allowed phages to sustain a gradual increase of flow rates from 0.5 to 3 lagoon volumes per hour over 10 days, with a phage wash-out starting to occur at higher flow rates. In the initial phase of PACE (first 100 hours), we modulated the selection pressure by fluctuating the dilution rates between 0.5 and 1 lagoon volume per hour (LV/h) in order to facilitate crossing of potential fitness valleys, followed by a more stringent selection with a dilution rate that was increased to 3 LV/h. To assess the shift of phage genotypes during PACE, we collected phage DNA at different timepoints during the experiment and analysed it by nanopore long read sequencing. Evaluation of the sequencing data revealed a mutation bias of G-C to A-T conversions (51.3%), with A-T to T-A conversions being the least frequently observed mutations (2.0%, n=762). Importantly, amino acid changes occurred most often in the RuvC III domain, phosphate lock loop domain, wedge domain and PAM interacting domain of *Cj*Cas9 (**Fig. 1c**). After 108 hours of PACE, phages with the consensus mutations L58Q, G323V, D376Y, F434C, K622N, D663E, T671A, E789K, N821K, D891N, S918T, S932N and S951G started to occur in the lagoon. These mutations were further enriched until termination of the experiment, where a consensus genotype made up the bulk of the phage.

*Establishment of an evolved CjCas9 variant with broad PAM recognition*

In our PACE and PANCE evolution logic we selected for PAM recognition via *Cj*Cas9 binding, potentially leading to mutations that impair the nuclease activity of Cas9. Hence, we first assessed the effect of the consensus mutations individually in a kinetic *in vitro* cleavage assay on a randomized PAM library, with the aim of finding a set of mutations that lead to PAM relaxation without impairing the nuclease activity. Of the 13 consensus mutations, E789K, E871K, D891N and E946K had a strong contribution to PAM relaxation compared to wild type *Cj*Cas9 at position 5 and 7 (**Fig. 2a**), with all 4 mutations leading to the recognition of non-canonical C and T bases at position 5 and non-canonical C, G and T bases at position 7. Since E789K resulted in a higher cleavage activity at most non-canonical PAMs than the other three variants, and additionally allowed pronounced recognition of $N_5$ACA PAMs, we decided to select this mutation for our evolved (evo)*Cj*Cas9 variant. Interestingly, mapping E789K onto the cryogenic electron microscopy (cryo-EM) structure of *Cj*Cas (Protein Data Bank (PDB): 5X2H) revealed that it is located in the phosphate lock loop domain and interacts with the phosphate backbone of PAM position 1. The positively charged lysine residue may therefore facilitate distortion of the target DNA strand, which in turn allows recognition of non-canonical bases at downstream PAM positions.

The mutation S951G occurred with the highest frequency of all PAM-relaxing consensus mutations (after 170h of PACE 93.9% of phage genomes contained S951G) and was the only mutation that allowed relaxation of PAM position 6, leading to the recognition of a non-canonical A nucleobase in addition to the canonical C and T. We therefore also selected this mutation for introduction into evo*Cj*Cas9. Notably, S951 is located in the PAM interacting domain, but instead of interacting with nucleobases at position 6 of the PAM it interacts with the nucleobases on the opposing DNA target strand via hydrogen bonding (Fig. 2b). We therefore speculate that the shorter side chain of glycine compared to serine reduces the interactions with A nucleobases at this position, leading to the detection of non-canonical T at position 6 of the PAM.

Substitutions of residues L58 and N821 were also frequently observed at the end of our PACE experiment (98.6% and 76.7%, respectively), but did not substantially contribute to PAM relaxation of *Cj*Cas9 in our PAM detection assay. Nevertheless, L58Y has previously been described to increase the stability of the *Cj*Cas9-sgRNA complex by forming a stacking interaction with U48 in the sgRNA, thereby enhancing the DNA cleavage activity of *Cj*Cas9 on canonical PAMs (**Fig. 2a**). Since N821 is in close physical distance and interacts with the same nucleotide of the sgRNA via hydrogen bonding (Fig. 2b), we speculate that the positively charged lysine residue at this position could further stabilize the *Cj*Cas9-sgRNA complex and enhance cleavage activity, prompting us to introduce L58Y and N821K into evo*Cj*Cas9.

Mutations G323V, D376Y, F434C, K622N, D663E and T671A were also enriched during PACE, but had a detrimental effect on the catalytic activity of *Cj*Cas9 nuclease in our cleavage assay. Thus, while these mutations may pose a fitness advantage regarding target recognition and binding of *Cj*Cas9, they would prohibit applications where cutting or nicking of DNA is required. We therefore did not integrate these mutations into evo*Cj*Cas9.

Taken together, we found that the mutations L58Y, E789K, N821K and S951G increase PAM flexibility or cleavage activity. Combined with D900K, a mutation that was not enriched in our PACE experiment but was previously described to enhance *Cj*Cas9 cleavage activity (Nakagawa, R. et al. Engineered Campylobacter jejuni Cas9 variant with enhanced activity and broader targeting range. Commun. Biol. 5, 1-8 (2022)), these mutations were introduced into *Cj*Cas9 to generate evo*Cj*Cas9. Compared to wild type *Cj*Cas9 and the previously established enhanced (en)*Cj*Cas9 variant with the substitutions L58Y and D900K, evo*Cj*Cas9 shows a substantial relaxation in PAM recognition (**Fig. 2a**); it recognizes all nucleotides at the first three PAM positions and NAHNN or NNHAN sequences at PAM positions 4-8. This allows evo*Cj*Cas9 to detect 10 times more target sequences than wild type *Cj*Cas9, which requires a VRYAC motif at PAM positions 4-8 (198 versus 19 PAM sequences with a kinetic rate constant $>10^{-3}$, **Fig. 2a**).

*evoCjCas9 shows high cleavage activity at canonical and non-canonical PAMs in human cells*

To comprehensively characterize the activity of evo*Cj*Cas9, we compared indel frequencies of *Cj*Cas9 and evo*Cj*Cas9 in mammalian cells at multiple sites with canonical and non-canonical PAM sequences. We therefore generated a lentiviral library containing 267 *Cj*Cas9 sgRNA constructs paired with their target sites (self-targeting library), with the different target sites comprising a set of canonical ($N_4ACAC$, $N_4ATAC$, $N_4GTAC$ and $N_4GCAC$) and non-canonical ($N_4AAAC$, $N_4CAAC$, $N_4GAAC$ and $N_4TAAC$) PAM sites. After integrating the library into the genome of HEK293T cells, plasmids expressing wild type *Cj*Cas9 or evo*Cj*Cas9 were transfected (**Fig. 3a**) and after selection genomic DNA was isolated and analysed by high throughput sequencing (HTS). Interestingly, at canonical PAM sites evo*Cj*Cas9 was already 2-fold more efficient than wild type *Cj*Cas9 in generating indels (on average 54.0% versus 24.7%, **Fig. 3b**). Moreover, evo*Cj*Cas9 also reached average editing rates of 48.6% at non-canonical PAM sites, at which wild type *Cj*Cas9 did not show substantial editing (**Fig. 3b**).

Since other PAM relaxed Cas9 variants often come with the trade-off of reduced activity, we next benchmarked indel formation rates of commonly used Cas9 orthologs (*Cj*Cas9, en*Cj*Cas9, *Sa*Cas9, *Sauri*Cas9, *Sp*Cas9 - **Fig. 3a**) and their PAM-relaxed variants (evo*Cj*Cas9, *Sa*KKH, *Sauri*Cas9-KKH, *Sp*RY, *Sp*G). We designed a self-targeting library consisting of 45 target sites, with each target site containing the optimal protospacer length and PAM sequence for each orthologue (22 bp and $N_3AACAC$ for *Cj*Cas9, 22 bp and $N_2GRRT$ for *Sa*Cas9, 22 bp and $N_2GG$ for SauriCas9, 19 bp and NGG for SpCas9). The library was cloned into a lentiviral plasmid and after virus production integrated into the genome of HEK293T cells. The cell pool was subsequently transfected with plasmids expressing the different Cas9 variants, and after selection genomic DNA was extracted for analysis by HTS. Confirming previous studies, we found that PAM relaxed *Sa*Cas9-, *Sauri*Cas9- and SpCas9 variants showed lower activity than the wild type orthologs on canonical PAMs (**Fig. 3c**). In contrary, evo*Cj*Cas9 showed substantially higher activity than wild type *Cj*Cas9 on the canonical $N_3AACAC$ PAM sequence, which could be explained by the activity enhancing mutations that are also present in en*Cj*Cas9 (**Fig. 3c**). Cross-comparison to other Cas9 orthologs further revealed that evo*Cj*Cas9 works with similar efficiency as *Sp*G, a frequently used *Sp*Cas9 variant detecting NGN PAM sequences that occur with similar frequencies than evo*Cj*Cas9 PAM sequences. Moreover, in our assay evo*Cj*Cas9 outperforms *Sp*RY, the most PAM relaxed SpCas9 variant that has been established so far (**Fig. 3c, d**).

*evoCjCas9 has a low tolerance for mismatches between the spacer and target site*

Off-target editing at genomic sites that share sequence similarities to the targeted locus are limiting clinical application of CRISPR-Cas nucleases. We therefore next assessed the mismatch tolerance of wild type and evo*Cj*Cas9. To this end, we designed a self-targeting library with 687 members, consisting of 37 target sites and up to 19 potential off-target sites with 1-5 mismatches. The library was stably integrated into HEK293T cells using lentiviral vectors and cells were transfected with plasmids expressing wild type- or evo*Cj*Cas9. HTS analysis of the target sites revealed that both variants have a similar mismatch tolerance; the mean off-target (1-5 mismatches) to on-target ratio for *Cj*Cas9 and evo*Cj*Cas9 was 0.36 and 0.39, respectively, with both variants not tolerating more than 2 mismatches between the spacer and protospacer (**Fig. 3e**). To further investigate how the location of the mismatches within the protospacer influences off-target editing activities, we next designed a dual-mismatch permutation library for 10 *Cj*Cas9 target sites. As expected from previous observations with *Sp*Cas9, mismatches in the seed region of the sgRNA (position 14-22) were more detrimental to target recognition than PAM distal mismatches (**Fig. 3f**).

*Efficient base- and prime editing with evoCjCas9*

The broad PAM recognition and high activity of evo*Cj*Cas9 makes it an ideal nuclease for generating size optimized BEs and PEs. To first assess base editing with evo*Cj*Cas9, we fused evo*Cj*Cas9- or wild type *Cj*Cas9 variants that nick the target strand (D8A) C-terminally to either an adenosine deaminase (ABE8e; Richter, M. F. et al. Phage-assisted evolution of an adenine base editor with improved Cas domain compatibility and activity. Nat. Biotechnol. 38, 883-891 (2020); WO2021151756A1, incorporated herein by reference) or to a cytosine deaminase (hsAIDmax (Komor et al. Programmable editing of a target base in genomic DNA without double-stranded DNA cleavage. Nature 533, 420-424 (2016)), eAID (Liu, Z. et al. Improved base editor for efficient editing in GC contexts in rabbits with an optimized AID-Cas9 fusion. 33, 9210-9219 (2019)), BE4max and AncBE4max (Koblan, L. W. et al. Improving cytidine and adenine base editors by expression optimization and ancestral reconstruction. Nat. Biotechnol. 36, 843-848 (2018))) (**Fig. 4a**). Cytosine BEs were furthermore N-terminally fused to an uracil glycosylase inhibitor (UGI) to block the conversion of the installed U back to a C. We next designed a self-targeting library consisting of 254 target sites with canonical and non-canonical PAM sites for base editing with CjCas9-BEs. The library was stably integrated into HEK293T cells using lentiviral vectors, which were subsequently transfected with plasmids expressing the different base editors. In line with our results obtained with the evo*Cj*Cas9 nuclease, evo*Cj*Cas9 BEs already showed higher editing efficiency than wild type *Cj*Cas9 BEs at canonical PAMs. In addition, evo*Cj*Cas9 BEs allowed base editing at non-canonical PAMs, where wild type *Cj*Cas9 BEs were largely inactive (**Fig. 4b**). Similar to previously reported BEs established from other compact Cas9 orthologues, *Cj*Cas9 BEs had a broader editing window than the typical editing window of *Sp*Cas9 BEs. We reasoned that this might be caused by a less constrained R-loop formation, and next replaced the HNH domain of evo*Cj*Cas9 with a heterodimer of TadA-TadA8e to constrain the deaminase as close to the R-loop as possible. However, while the editing window of HNHxevo*Cj*Cas9 was indeed more constrained, similar to reports with HNHxSpCas9 BEs, editing rates were also substantially lower than for *Cj*Cas9 BEs with N-terminally fused TadA8e (**Fig. 4b,** last panel). Finally, we also assessed if *Cj*Cas9 base editors show preferences for certain trinucleotide motifs (bases flanking the targeted A or C). Consistent with recent reports for *Sp*Cas9 Bes, we found that evo*Cj*Cas9 BEs using the rAPOBEC1 deaminase show a preference for editing of C bases that are preceded by a T, while evo*Cj*Cas9 BEs using AID show a preference for editing of C bases that are preceded by A or G. In addition, we observed that evo*Cj*Cas9 BEs using the TadA8e deaminase largely disfavor target bases preceding A.

Prime editing is a more versatile genome editing technology than base editing that enables the introduction of all 12 possible base conversions as well as multi base replacements and small insertions or deletions. To generate miniature PE systems based on *Cj*Cas9, we first introduced the H559A mutation into wild type *Cj*Cas9 and evo*Cj*Cas9 to obtain variants that only nick the non-target strand. These variants were then N-terminally fused to either the full length M-MLV reverse transcriptase (PE) or an M-MLV reverse transcriptase that lacks the RNaseH domain (PE$^{\Delta RnH}$) (**Fig. 4c**). When we tested these variants by introducing an A to G mutation into the AAVS1 locus, we observed that wild type *Cj*Cas9 PEs only achieved 6% editing while evo*Cj*Cas9 PEs achieved up to 26% editing, with no significant difference between full length PE and PE$^{\Delta RnH}$ (Fig. 4d). We next generated a self-targeting library containing 236 prime editing guide (peg)RNAs paired with their target sites. These target sites include human disease loci with canonical- and non-canonical PAMs, which are corrected back to the wild type sequence by their corresponding pegRNAs. The library was integrated into HEK293T cells using lentiviral vectors, and cells were subsequently transfected with plasmids expressing *Cj*Cas9-PE$^{\Delta RnH}$ or evo*Cj*Cas9-PE$^{\Delta RnH}$. In line with the results from the AAVS1 locus, evo*Cj*Cas9-PE$^{\Delta RnH}$ already achieved higher editing rates (up to 30.1 %) than *Cj*Cas9-PE$^{\Delta RnH}$ (up to 24.6%) at target sites with canonical PAM sequences. In addition, evo*Cj*Cas9-PE$^{\Delta RnH}$ also enabled editing at target sites with non-canonical PAMs (up to 22%), whereas wild type *Cj*Cas9-PE$^{\Delta RnH}$ was unable to edit these sites (Fig. 4e;). The same pattern was also observed when the library was integrated in K562 cells, albeit with lower overall editing rates. Further analysis of *Cj*Cas9 prime editing outcomes on the library revealed that single nucleotide replacements were more efficient than insertions or deletions, with A to C and C to T conversions being the most efficient edits. Notably, within our library we also observed multiple loci with low or no prime editing, suggesting that at these sites our pegRNA design was not optimal (Fig. 4f). We therefore next assessed if we could identify pegRNA designs that correlate with editing rates in our library (n=238). We found that the most influential features contributing to *Cj*Cas9 prime editing were the melting temperature of the primer binding sequence (PBS) (R=0.43) and the reverse transcriptase template (RTT) (R=0.26, suggesting that extending the pegRNA length downstream of the scaffold sequence may benefit editing rates.

*In vivo base editing with single AAV constructs*

The small size of evo*Cj*Cas9 compared to other Cas9 orthologs offers major advantages for *in vivo* genome editing, and in principle facilitates packaging of evo*Cj*Cas9 nucleases and evo*Cj*Cas9 BEs together with their sgRNAs on single AAV vectors. To test whether this approach is feasible and enables efficient editing *in vivo,* we cloned an AAV construct that expresses evo*Cj*Cas9-ABE8e under the hepatocyte specific P3 promoter and an sgRNA targeting the AG splice acceptor site of the *Pcsk9* intron 3 under the U6 promoter (**Fig. 5a**). Pcsk9 is a secreted protein that is expressed in the liver and that acts as a negative regulator of the low-density lipoprotein (LDL) receptor; reducing Pcsk9 levels by installing splice site mutations is therefore a potential therapeutic approach for reducing blood LDL levels. We packaged the evo*Cj*Cas9 BE expression construct spanning 4988 bp including ITRs into hepatotropic AAV9 capsids, which were systemically administered into 5-week-old C57BL/6J mice at a dose of $2.5 \times 10^{13}$ or $5 \times 10^{13}$ vector genomes (vg)/kg body weight (**Fig. 5b**). 6 weeks post injection we isolated liver tissue, hepatocytes and serum from treated mice to analyze editing rates and the effect on serum Pcsk9 and LDL levels. HTS on genomic DNA isolated from liver tissue revealed 22% and 21% editing in animals treated with the lower- and higher dose, respectively (**Fig. 5c**). In isolated hepatocytes, editing rates were substantially higher, with 38% and 39%, confirming hepatocyte-specific expression of evo*Cj*Cas9-ABE8e from the P3 promoter. Analysis of the serum of treated mice further revealed a 71% reduction of Pcsk9 levels and a 35% and 43% reduction of LDL levels in animals injected with a dose of $2.5 \times 10^{13}$ vg/kg and $5 \times 10^{13}$ vg/kg, respectively (**Fig. 5d, e**). Together, these data demonstrate that evo*Cj*Cas9 can be employed for efficient and tissue specific *in vivo* base editing from single AAV vectors at clinically relevant doses.

## DISCUSSION

The use of compact Cas9 orthologs instead of the 1,368 aa long *Sp*Cas9 nuclease is an attractive alternative for translational genome editing, as their small size facilitates *in vivo* delivery. With 984 aa, *Cj*Cas9 is the smallest known Cas9 ortholog, which in principle fits as a nuclease or a BE fusion protein together with its sgRNA on a single AAV vector. However, similar to other small-sized Cas9 orthologs, wild type *Cj*Cas9 recognizes a relatively long PAM, reducing the number of targetable sites. Using phage-assisted evolution (PACE and PANCE), we identified a set of amino acid substitutions that led to PAM relaxation of *Cj*Cas9.

The novel variant, termed evo*Cj*Cas9, recognizes $N_4$AH and NsHA PAM sequences, which occur 10 times more frequently in the genome than the canonical $N_3$VRYAC PAM site. In addition, evo*Cj*Cas9 possesses a nuclease activity that is higher than that of wild type *Cj*Cas9 (2-fold), and in range with the nuclease activity of PAM-relaxed *Sp*Cas9 variants such as *Sp*G. Moreover, the requirement of a 22 to 24 nucleotide long spacer sequence with a mismatch tolerance of less than 3 bases makes evo*Cj*Cas9 a highly precise targeted nuclease.

By fusing deaminases or the M-MLV reverse transcriptase to D8A or H559A evo*Cj*Cas9 nickase variants we also constructed compact base- and prime editors. Analyzing evo*Cj*Cas9 BEs, we observed average editing rates above 20% for various cytidine and adenine BE architectures, albeit with wider editing windows than *Sp*Cas9 BEs. Similar observations have been made with BEs that were based on other compact Cas9 proteins, such as *Sa*Cas9 and *Nme*2Cas9, suggesting differences in the R-loop accessibility between *Sp*Cas9 and smaller Cas9 orthologs. While with evo*Cj*Cas9 PEs we also achieved >20% editing at certain target sites, on average the editing rates were lower than for evo*Cj*Cas9 BEs. Optimizing linker architectures or the *Cj*Cas9 pegRNA design may therefore be required to obtain robust editing at a larger number of loci.

Due to the small size of evo*Cj*Cas9 it can be packaged as an adenine- or cytosine BE on a single AAV vector. While adenine base editing from single AAV vectors has already been demonstrated previously with *Cj*3Cas9, *Sa*Cas9 and *Nme*2Cas9, to our knowledge this study provides the first demonstration for cytosine base editing from a single AAV vector. Furthermore, the compact size of *Cj*Cas9 would also allow for expression of ABEs using tissue-specific promoters other than the demonstrated P3 and hSyn promoters. Examples include the hUPII (bladder; urothelium), ProA7 (eye; cone cells), Ksp-cadherin (kidney; renal tubules), $\alpha$-MHC (cardiac muscle), CK8e or SPc5-12 (skeletal muscle) promoters.

Taken together, we used PACE and PANCE to develop evo*Cj*Cas9, a compact Cas9 nuclease with a broad targeting range and high nuclease activity. evo*Cj*Cas9 possesses a high specificity and enables base- and prime editing in mammalian cells. Due to its small size, we could also develop a single vector AAV system for *in vivo* adenine base editing from tissue-specific promoters, demonstrating the potential of evo*Cj*Cas9 for future therapeutic applications.

## METHODS

**General methods and cloning.** PCRs were performed using the Q5 High-Fidelity DNA polymerase. All expression vectors were assembled using NEBuilder HiFi DNA assembly. Plasmids expressing sgRNAs were cloned using the T4 DNA ligase (New England Biolabs). Plasmids used in mammalian tissue culture were purified using NucleoBond Xtra Midi kits (Macherey-Nagel).

**Phage-assisted continuous and non-continuous evolution.** PANCE and PACE were performed as recently described (Hu, J. H. et al. Evolved Cas9 variants with broad PAM compatibility and high DNA specificity. Nature 556, 57-63 (2018); Miller, S. M., Wang, T. & Liu, D. R. Phage-assisted continuous and non-continuous evolution. Nat. Protoc. 15, 4101-4127 (2020)). The M13 filamentous selection phage encodes a catalytically dead *Cj*Cas9 fused via an Ala-Ala-Linker to the $\omega$ subunit of bacterial RNA polymerase. The accessory plasmid harbors the PAM and protospacer sequence in reverse, 114 nt upstream of gene III. To increase the mutagenesis rate, DP6 (Addgene #140446) was used and induced with L-arabinose (10 mM) one hour before selection phage addition. All experiments were performed using the S2060 bacterial strain (Addgene #105064), cultured in Davis Rich Media. PANCE was performed using PAM libraries with all possible nucleobase combinations for either PAM positions 5, 6, 7, or 8. PANCE lagoons were contained in deep well plates at 1 mL volume and selection phages were added once cultures reached the log phase (approx. OD 0.4 - 0.6). Phages were incubated overnight and harvested via centrifugation and sterile filtration (0.2 $\mu$m, Sarstedt) before adding a dilution to the next round. Phage titers were determined using plaque assays or RT-qPCR for higher throughput. qPCR results with CT > 30 were considered to resemble the absence of phages. Similarly, PACE was performed using a pool of PANCE-derived phage variants after round 12, mixed 1:1 with wild type *Cj*Cas9 containing phages. As accessory plasmid, a PAM library for positions 6-8 was used. S2060 host cells were continuously cultured at OD 0.5 in a custom-built miniature bioreactor. Host cells were pumped through a lagoon and the mutagenesis plasmid DP6 was induced with L-arabinose (10mM) before the cells reached the lagoon. The flow through the lagoon was controlled via a microcontroller and adjusted to increase the selection pressure over time. Phage titers in the bioreactor (no phages detected) and lagoon were monitored using RT-qPCR and plaque assays. Regular time points were collected and analysed using long-read Nanopore sequencing (Oxford Nanopore Technologies).

**Phylogenetic analysis of directed evolution outcomes.** Phage genotypes emerging during PACE were sequenced using a previously established method. Briefly, from collected phages at different time points $\omega$ subunit-*Cj*Cas9 variants were PCR amplified using unique molecular identifiers (UMIs) and timepoint specific barcodes and were subcloned. Per timepoint, a few hundred colonies were selected and clonally amplified. Plasmids were purified and $\omega$ subunit-CjCas9 was extracted using unique restriction sites. Nanopore adapters were ligated, and variants were sequenced using the manufacturer's protocol. Timepoints were demultiplexed and consensus reads for each UMI were built using a python script.

**High-throughput PAM detection assay.** HT-PAMDA was performed as recently described (Walton et al. Scalable characterization of the PAM requirements of CRISPR-Cas enzymes using HT-PAMDA. Nat. Protoc. 16, 1511-1547 (2021). Cas9-variants were cloned in pCMV-T7-SpCas9-P2A-EGFP (Addgene #139987) and expressed in HEK293T cells for 48 hours. Whole-cell lysates were collected and normalized to a concentration corresponding to 150 nM fluorescein dye. Target-specific sgRNAs were *in vitro* transcribed using HiScribe T7 High Yield RNA Synthesis Kit. Substrate libraries with different target sites and PAM libraries were cloned into p11-LacY-wtxq (Addgene #69056). gRNA (1.1 $\mu$M) and normalized cell lysate (83 nM fluorescein) were complexed for 10 minutes at 37 °C. The RNP mixture (0.5 $\mu$M gRNA, 37.5 nM fluorescein lysate) was added to the substrate library (2.5 nM) and the reaction was stopped after different time intervals (1, 8, and 32 minutes). For all time points, substrate libraries were individually PCR amplified using timepoint-specific barcodes, followed by an amplification using protein variant-specific barcodes. Samples were pooled and sequenced on a NovaSeq 6000 (Illumina). Cas9-variants were characterized on two different target sites, with the PAM library spanning either positions 1 to 5 (Spacer 3) or 3 to 8 (Spacer 1 and 2). Two replicates were performed on different days. Sequencing data were processed using the provided python script. Rate constants were calculated from normalized read counts for all characterized CjCas9 variants.

**Target library design using sgRNAs.** Self-targeting constructs were designed with guide sequence preceding 5'-G nucleotide (not counted to total guide length) and ordered as single-stranded DNA oligo pools (Twist Bioscience). Oligo pools were amplified according to the manufacturer's protocols using NEBNext Ultra II polymerase. The amplified pool was cloned into BsmBI-digested Lenti_gRNA-Puro (Addgene #84752) and electroporated into Endura electrocompetent cells (Lucigen). Colonies were harvested (> 2000x coverage) and plasmids were purified.

**Target library screen using pegRNAs.** A recently developed prime editing prediction tool (PRIDICT, unpublished) was adapted to assist in the design of the pegRNAs for the library. The library was cloned at > 2000x coverage as described above. Upon harvest, plasmids were digested using BsmBI, purified, and the *Cj*Cas9 scaffold sequence was inserted via golden gate assembly. Purified ligation reactions were electroporated, colonies were harvested (> 2000x coverage) and plasmids were purified.

**Cell culture and high-throughput sequencing.** All cell lines were cultured at 37°C and 5% $CO_2$ in cell culture incubators. HEK293T cells (ATCC CRL-3216) were maintained in Dulbecco's modified Eagle's medium (DMEM) plus GlutaMAX (Thermo Fisher Scientific), and K562 (ATCC CCL-243) cells were maintained in RPMI-1640 medium (Thermo Fisher Scientific), both supplemented with 10% (v/v) fetal bovine serum (FBS; Sigma-Aldrich) and 1% penicillin/streptomycin (Thermo Fisher Scientific). Cells were maintained at confluency below 90% and depending on library size seeded on 6-well/48-well cell culture plates or T175 cell culture flasks (Greiner). For base editor target library screens, HEK293T cells were transfected at 70% confluency (day 0) using 10 $\mu$l Lipofectamine 2000 (Thermo Fisher Scientific), 3 $\mu$g of base editor expression plasmid and 1 $\mu$g of Tol2-helper plasmid (Addgene #31828) according to the recommendation of the manufacturer.

Prime editing library screens in HEK293T were performed in T25 flasks by transfection of 2.64 $\mu$g of editor plasmid with OptiMEM (total volume of 75.14 $\mu$l) and 21.7 $\mu$l polyethylenimine (PEI, 1 mg/ml) (Day 0). PE screens in K562 were performed in 48-well plates (16x lipofection per editor or control) by seeding 100,000 K562 cells in 300 $\mu$l

RPMI++ and transfection of 1000 ng editor plasmid with OptiMEM (total volume of 25 μl) mixed with 1.5 μl of Lipofectamine 2000 (Thermo Fisher Scientific) and 23.5 μl of OptiMEM. Controls for HEK293T and K562 were transfected without editor plasmid. Arrayed editing experiments in HEK293T were performed in 48-well plates by seeding 130,000 cells 6h before transfection. Transfection was performed with 750 ng of editor plasmid and 250 ng of gRNA plasmid with 1 μl of Lipofectamine 2000 as described above for K562 (day 0). On day 1, HEK293T (detached using 1x TrypLE (Gibco)) or K562 cells were transferred into blasticidin (7.5 mg/mL) containing media (no selection for controls) and maintained for 6 (PE) or 9 (BE) more days (unless otherwise noted).

Genomic DNA was isolated by direct lysis and locus-specific primers were used to generate targeted amplicons (NEBNext Ultra II) for deep sequencing, maintaining > 500x coverage. Cells were tested negative for Mycoplasma contamination and were authenticated by the supplier by STR analysis.

**Lentiviral integration of self-targeting libraries.** For lentivirus production, HEK293T cells were seeded at $15 \times 10^6$ cells in a T175 flask (Greiner) and transfected at 70% confluency using polyethylenimine (PEI). Briefly, 152 μl PEI (0.1 mg/ml) was mixed with 506 μl Opti-MEM containing 2.6 μg VSV-G (Addgene #12259), 5.2 μg PAX2 (Addgene #12260) and 10.4 μg lentiviral vector plasmid. The mix was incubated for 20 min at room temperature prior to transfection. One day post-transfection, media was replaced and three days post-transfection, the supernatant was harvested and purified by filtration (0.4 μm, Sarstedt). A dilution series of produced lentivirus was used to transduce HEK293T cells. 24 hours post-transduction, cells were selected with 2.5 μg/ml puromycin. Lentiviral concentration with a 50% survival rate 3 days post-transduction was used for subsequent library cell generation. Library cells were generated accordingly, with cell numbers being sufficient for > 2000x coverage (post-selection). 3 days post-transduction, puromycin-containing media was replaced, and 5 days post-transduction library cells were detached and cryopreserved.

**Expression and purification of CjCas9 and evoCjCas9.** DNA sequences encoding CjCas9 and evoCjCas9 were inserted into the 1S plasmid (Addgene #29659) using ligation-independent cloning, resulting in a construct comprising a N-terminal His6-SUMO tag followed by a tobacco etch virus (TEV) protease cleavage site. The constructs were expressed in E. coli BL21 Rosetta2 (DE3) cells (Novagen). Cells were lysed in 20 mM Tris-HCl pH 8.0, 1 M NaCl, 20 mM imidazole, 1 μg/mL pepstatin, 200 μg/mL 4-(2-Aminoethyl) benzenesulfonyl fluoride hydrochloride (AEBSF), 1 mM Tris-(2-Carboxyethyl)phosphine hydrochloride (TCEP) by ultrasonication. Clarified lysate was loaded on a 5 mL Ni-NTA (Sigma-Aldrich) affinity column. The column was washed with 20 mM Tris-HCl pH 8.0, 1 M NaCl, 20 mM imidazole, 1 mM TCEP and bound protein was eluted with 20 mM Tris-HCl pH 8.0, 300 mM NaCl, 300 mM imidazole, 1 mM TCEP. The eluted protein was dialyzed, overnight at 4°C, against a buffer yielding a final imidazole concentration of 40 mM imidazole, in the presence of 1 mM TCEP and TEV protease to remove the His6-SUMO tag. Cleaved protein was passed through a 5 mL Ni-NTA (Sigma-Aldrich) affinity column to remove the cleaved affinity tag and uncleaved protein; the column was washed with 20 mM Tris-HCl pH 8.0, 300 mM NaCl, 40 mM imidazole, 1 mM TCEP, and the flow-through and wash fractions were pooled for subsequent purification using a 5 mL HiTrap HP Heparin column (Cytiva), eluting with a linear gradient up to 1 M NaCl. The elution fractions were pooled, concentrated in the presence of 1 mM Dithiothreitol (DTT) and further purified by size exclusion chromatography using a Superdex 200 (16/600) column (Cytiva) in 20 mM Tris-HCl pH 8.0, 150 mM NaCl, 1 mM DTT, yielding pure, monodispersed proteins. Aliquots were flash-frozen in liquid nitrogen and stored at -80 °C.

**CHANGE-seq.** The sgRNAs were first tested for functionality by digesting a PCR amplicon of the genomic target site. The library was prepared as previously described44. Data were processed using the CHANGE-seq analysis pipeline (https://github.com/tsailabSJ/changeseq) with the following parameters: 'read_threshold: 4, window_size: 3, mapq_threshold: 50, start_threshold: 1, Gap_threshold: 3, mismatch_threshold: 10, search_radius: 30, merged_analysis: True. The respective target sites were deep sequenced and covered by at least 10,000 reads per site.

**Adeno-associated virus production.** Vectors (AAV2 serotype 9) were produced by the Viral Vector Facility of the Neuroscience Center Zurich. Briefly, AAV vectors were ultracentrifuged and diafiltered. Physical titers (vg/mL) were determined using a Qubit 3.0 fluorometer as previously described. Identity of the packaged genomes of each AAV vector were confirmed as size verification of PCR amplicon (gel electrophoresis) and Sanger sequencing.

**Animal studies.** Mouse experiments were performed in accordance with protocols approved by the Kantonales Veterinäramt Zürich. Mice were housed in a pathogen-free animal facility at the Institute of Pharmacology and Toxicology at UZH Zurich and kept in a temperature- and humidity-controlled room (21°C, 50% RH) on a 12-h light/dark cycle. Mice were fasted for 3-4 h before blood was collected from the inferior vena cava before liver perfusion. Mice were injected with $0.5\text{-}1 \times 10^{12}$ AAV vector genomes per mouse. Injection volumes were 120-150 μl. Only male C57BL/6J animals at an age of 5-6 weeks were used.

**Primary hepatocyte isolation.** Mice were euthanized using $CO_2$ and immediately perfused with Hank's balanced salt solution (Thermo Fisher Scientific) plus 0.5 mM EDTA via the inferior vena cava and a subsequent incision in the portal vein. During this step, one liver lobe was squeezed off via a thread to inhibit perfusion of this lobe to collect whole liver samples for whole liver lysates. After blanching of the liver, mice were perfused with digestion medium (low-glucose DMEM plus 1× penicillin-streptomycin (Thermo Fisher Scientific), 15 mM HEPES and freshly added Liberase (Roche)) for 5 min. Livers were isolated in cold isolation medium (low-glucose DMEM supplemented with 10% (vol/vol) FBS plus 1× penicillin-streptomycin (Thermo Fisher Scientific) and GlutaMax (Thermo Fisher Scientific)), and the liver was gently dissociated to yield a cell suspension that was passed through a 100-$\mu$m filter. The suspension was then centrifuged at 50g for 2 min and washed with isolation medium 2-3 times until the supernatant was clear. The primary hepatocytes were pelleted for direct lysis for NGS sample preparation.

**ELISAs.** Mouse PCSK9 levels were determined by using Mouse Proprotein Convertase 9/PCSK9 Quantikine ELISA Kit (R&D Systems, cat. no. MPC900) according to the manufacturer's instructions. Absorbance was measured at 450 nm and background at 540 nm; the latter was subtracted for quantification. Total cholesterol, triglyceride and high-density lipoprotein (HDL) from all mouse samples were measured as routine parameters at the Division of Clinical Chemistry and Biochemistry at the University Children's Hospital Zurich using Alinity ci-series. LDL levels were calculated by using the Friedewald formula.

**HTS data analysis.** Libraries were sequenced on a MiSeq or NovaSeq 6000 (Illumina, 150bp, paired-end). Amplicon sequences were analyzed using custom Python scripts or aligned to their reference sequences using CRIPResso2. In brief, editing rates for BE and PE libraries were determined by calculating the ratio of edited bases (BE) or sequence-stretches (PE; 2bp before nick until 5bp after flap) and subsequently correcting editing rates with background rates from control samples as previously described (PRIDICT, unpublished).

**Claims**

1. A *Cj*Cas9 variant of SEQ ID NO 001, **characterized by** at least one of the following mutations: E789K and/or S951G.

2. The *Cj*Cas9 variant according to claim 1, further comprising the mutation D900K or D900R, particularly D900K.

3. The *Cj*Cas9 variant according to any one of the preceding claims, further comprising a substitution of L58 by Y or Q, particularly L58Y.

4. The *Cj*Cas9 variant according to any one of the preceding claims, further comprising a substitution of N821, particularly N821K.

5. The *Cj*Cas9 variant according to any one of the preceding claims, comprising both E789K and S951G.

6. The *Cj*Cas9 variant according to any one of the preceding claims, further comprising a substitution selected from the group of G323V, D376Y, F434C, K622N, D663E and T671A.

7. The *Cj*Cas9 variant according to any one of the preceding claims, further comprising a substitution selected from E871K, D891N and E946K.

8. The *Cj*Cas9 variant according to any one of the preceding claims, comprising the sequence of SEQ ID NO 002, or a sequence having at least 95% identity to SEQ ID NO 002 and bearing the mutations L58Y, E789K, N821K, D900K, S951G, and having a less stringent PAM requirement than SEQ ID NO 001.

9. A polypeptide comprising a *Cj*CAS9 variant according to any one of the preceding claims, wherein the polypeptide further comprises a nucleotide deaminase activity.

10. The polypeptide according to claim 9, wherein the deaminase is selected from: an adenosine deaminase activity; or a cytidine deaminase activity.

11. A polypeptide comprising a CjCAS9 variant according to any one of the preceding claims 9 to 11, wherein the polypeptide further comprises a reverse transcriptase activity.

12. The polypeptide according to claim 11, wherein the polypeptide does not comprise an RNAseH activity.

13. A polynucleotide sequence encoding a *CJ*Cas9 variant or a polypeptide comprising a *Cj*CAS9 variant, as specified in any one of the preceding claims.

14. An expression vector comprising a polynucleotide according to claim 13, under control of a promoter.

15. The expression vector according to claim 14, wherein the expression vector is an AAV virus particle.

Fig. 1

a

b

Fig. 1 (continued)

c

Fig. 2

a

Fig. 3

a

Fig. 3 (continued)

b

c

d

Fig. 3 (continued)

e

f

Fig. 3 (continued)

g

Fig. 4

a

b

Fig. 4b (continued)

**hsAID**

**eAID**

**TadCDd**

**ABE8e**

C

Fig. 4 (continued)

d

e

f

Fig. 5

a

b

c

d

Fig. 5 (continued)

Fig. 6

Fig. 6 (continued)

Fig. 7

Fig. 8

a

b

Fig. 9

a

Fig. 9 (continued)

b

Fig. 10

Fig. 11

Fig. 12

| | Europäisches Patentamt European Patent Office Office européen des brevets | **EUROPEAN SEARCH REPORT** | Application Number EP 23 20 3923 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBLWGS [Online]<br><br>2 September 2020 (2020-09-02),<br>"Campylobacter jejuni type II CRISPR RNA-guided endonuclease Cas9",<br>XP093137641,<br>retrieved from EBI<br>Database accession no. EAI4715943<br>* abstract *<br>----- | 1,13 | INV.<br>C12N9/22<br>C12N15/62 |
| X | DATABASE UniProt [Online]<br><br>3 May 2023 (2023-05-03),<br>"RecName: Full=CRISPR-associated endonuclease Cas9<br>{ECO:0000256\|HAMAP-Rule:MF_01480};<br>EC=3.1.-.-<br>{ECO:0000256\|HAMAP-Rule:MF_01480}",<br>XP093137624,<br>retrieved from EBI<br>Database accession no. A0A690ANS8<br>* the whole document *<br>----- | 1,5,13 | |
| X | DATABASE UniProt [Online]<br><br>2 May 2023 (2023-05-02),<br>"RecName: Full=CRISPR-associated endonuclease Cas9<br>{ECO:0000256\|HAMAP-Rule:MF_01480};<br>EC=3.1.-.-<br>{ECO:0000256\|HAMAP-Rule:MF_01480}",<br>XP093137623,<br>retrieved from EBI<br>Database accession no. A0A3S4TLQ3<br>* the whole document *<br>-----<br>-/-- | 1,5,13 | TECHNICAL FIELDS SEARCHED (IPC)<br><br>C12N<br>C07K |

The present search report has been drawn up for all claims

| Place of search | Date of completion of the search | Examiner |
|---|---|---|
| The Hague | 5 March 2024 | Wiame, Ilse |

EPO FORM 1503 03.82 (P04C01)

page 1 of 2

Europäisches
Patentamt
European
Patent Office
Office européen
des brevets

**EUROPEAN SEARCH REPORT**

**Application Number**

**EP 23 20 3923**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | NAKAGAWA RYOYA ET AL: "Engineered Campylobacter jejuni Cas9 variant with enhanced activity and broader targeting range", COMMUNICATIONS BIOLOGY, vol. 5, no. 1, 8 March 2022 (2022-03-08), XP093137526, ISSN: 2399-3642, DOI: 10.1038/s42003-022-03149-7 Retrieved from the Internet: URL:https://www.nature.com/articles/s42003-022-03149-7> * the whole document * | 2-4, 6-12,14, 15 | |
| A | YAMADA MARI ET AL: "Crystal Structure of the Minimal Cas9 fromCampylobacter jejuniReveals the Molecular Diversity in the CRISPR-Cas9 Systems", MOLECULAR CELL, vol. 65, no. 6, 16 March 2017 (2017-03-16), page 1109, XP029959270, ISSN: 1097-2765, DOI: 10.1016/J.MOLCEL.2017.02.007 * the whole document * | 1-15 | |
| X,P | SCHMIDHEINI LUKAS ET AL: "Continuous directed evolution of a compact CjCas9 variant with broad PAM compatibility", NATURE CHEMICAL BIOLOGY, vol. 20, no. 3, 21 September 2023 (2023-09-21), pages 333-343, XP093137607, New York ISSN: 1552-4450, DOI: 10.1038/s41589-023-01427-x Retrieved from the Internet: URL:https://www.nature.com/articles/s41589-023-01427-x> * the whole document * | 1-15 | TECHNICAL FIELDS SEARCHED (IPC) |

The present search report has been drawn up for all claims

| Place of search | Date of completion of the search | Examiner |
|---|---|---|
| The Hague | 5 March 2024 | Wiame, Ilse |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2022045169 A1
- WO 2021151756 A1

### Non-patent literature cited in the description

- **NAKAGAWA et al.** *Communications Biology,* 2022, vol. 5 (211
- **RICHTER et al.** *Nat Biotechnol.,* July 2020, vol. 38 (7), 883-891
- **YAN.** *Molecular Plant,* 2021, vol. 14, 722-731
- **NEUGEBAUER et al.** *Nat Biotechnol,* 2022, https://doi.org/10.1038/s41587-022-01533-6
- **WALTON.** *Nat. Protoc.,* 2021
- *Commun. Biol.,* 2022, vol. 5, 1-8
- **RICHTER, M. F. et al.** Phage-assisted evolution of an adenine base editor with improved Cas domain compatibility and activity. *Nat. Biotechnol.,* 2020, vol. 38, 883-891
- **KOMOR et al.** Programmable editing of a target base in genomic DNA without double-stranded DNA cleavage. *Nature,* 2016, vol. 533, 420-424
- **LIU, Z. et al.** *Improved base editor for efficient editing in GC contexts in rabbits with an optimized AID-Cas9 fusion.,* 2019, vol. 33, 9210-9219
- **KOBLAN, L. W. et al.** Improving cytidine and adenine base editors by expression optimization and ancestral reconstruction. *Nat. Biotechnol.,* 2018, vol. 36, 843-848
- **HU, J. H. et al.** Evolved Cas9 variants with broad PAM compatibility and high DNA specificity. *Nature,* 2018, vol. 556, 57-63
- **MILLER, S. M. ; WANG, T. ; LIU, D. R.** Phage-assisted continuous and non-continuous evolution. *Nat. Protoc.,* 2020, vol. 15, 4101-4127
- *Nat. Protoc.,* 2021, vol. 16, 1511-1547